# EUROPEAN PATENT APPLICATION

(11) **EP 4 461 720 A1**
(43) Date of publication of application: **13.11.2024**
(21) Application number: 23737314.7
(22) Date of filing: 06.01.2023
(51) Int. Cl.: C07C 4/22, C07C 13/12, C08C 19/08, C08F 32/08, C07B 61/00

(54) **CYCLOPENTENE RECOVERY METHOD**

(30) Priority: 07.01.2022 JP 2022001909
(71) Applicant: Zeon Corporation, Tokyo 100-8246 (JP)
(72) Inventor: KUMAZAWA, Kazuki, Tokyo 100-8246 (JP); HAYANO, Shigetaka, Tokyo 100-8246 (JP)
(74) Representative: Maiwald GmbH
(86) International application number: PCT/JP2023/000195
(87) International publication number: WO 2023/132366

(57) **Abstract**

Provided is a method for recovering cyclopentene, comprising allowing a metathesis catalyst to act on a ring-opened polymer having a structural unit derived from cyclopentene to recover cyclopentene.

## Description

### TECHNICAL FIELD

The present invention relates to a method for recovering cyclopentene, and relates to a method for recovering cyclopentene that can effectively recover cyclopentene from a ring-opened polymer having a structural unit derived from cyclopentene.

### BACKGROUND ART

A ring-opened polymer having a structural unit derived from cyclopentene obtained by ring-opening polymerization of cyclic olefins containing cyclopentene is widely known as a rubber material, and is combined with an inorganic filler such as carbon black or silica and used as a rubber material for tires (see Patent Document 1). On the other hand, recently, from the viewpoint of resource depletion and environmental protection, chemical recycling has gained attention, and chemical recycling of various products containing such a ring-opened polymer having a structural unit derived from cyclopentene after use has been examined.

### RELATED ART DOCUMENT

### PATENT DOCUMENT

Patent Document 1: JP 2010-37362 A

### SUMMARY OF THE INVENTION

### PROBLEMS TO BE SOLVED BY THE INVENTION

In view of the above issue, the present invention is aimed at providing a method for recovering cyclopentene that can effectively recover cyclopentene from a ring-opened polymer having a structural unit derived from cyclopentene.

### MEANS FOR SOLVING PROBLEMS

As a result of extensive studies to achieve the above object, the present inventors have found that cyclopentene can be effectively recovered by allowing a metathesis catalyst to act on a ring-opened polymer having a structural unit derived from cyclopentene, and have completed the present invention.

That is, the present invention provides a method for recovering cyclopentene, comprising allowing a metathesis catalyst to act on a ring-opened polymer having a structural unit derived from cyclopentene to recover cyclopentene.

In the method for recovering cyclopentene according to the present invention, the ring-opened polymer is preferably a cyclopentene homopolymer or a copolymer of cyclopentene and a norbornene compound.

In the method for recovering cyclopentene according to the present invention, the ring-opened polymer is preferably a copolymer of cyclopentene and a norbornene compound.

In the method for recovering cyclopentene according to the present invention, the ring-opened polymer is preferably a random copolymer of cyclopentene and a norbornene compound.

In the method for recovering cyclopentene according to the present invention, the ring-opened polymer is preferably a cross-linked polymer.

In the method for recovering cyclopentene according to the present invention, cyclopentene is preferably recovered by allowing the metathesis catalyst to act on the ring-opened polymer in a solvent while controlling the concentration of cyclopentene to be in a range of 1.7 mol/L or less.

In the method for recovering cyclopentene according to the present invention, cyclopentene is preferably recovered by allowing the metathesis catalyst to act on the ring-opened polymer in a solvent while controlling the amount of the ring-opened polymer used such that the concentration of polycyclopentene repeating units in the ring-opened polymer is 5 mol/L or less.

The present invention also provides a cyclopentene solution containing cyclopentene and a reaction residue of a metathesis catalyst in a solvent, wherein the concentration of cyclopentene is 0.01 to 1.7 mol/L.

### EFFECTS OF THE INVENTION

The present invention can provide a method for recovering cyclopentene that can effectively recover cyclopentene from a ring-opened polymer having a structural unit derived from cyclopentene, and a cyclopentene solution suitably obtained by the method for recovering cyclopentene.

### DESCRIPTION OF EMBODIMENTS

The method for recovering cyclopentene according to the present invention is a method for recovering cyclopentene by allowing a metathesis catalyst to act on a ring-opened polymer having a structural unit derived from cyclopentene.

### <Ring-opened polymer>

The ring-opened polymer used in the present invention may be any ring-opened polymer having a structural unit derived from cyclopentene, and is not particularly limited. The ring-opened polymer used in the present invention may be a cyclopentene homopolymer, or may be a copolymer of cyclopentene and a monomer that can co-polymerize with cyclopentene. When the ring-opened polymer used in the present invention is a copolymer, it may be a random copolymer, or may be a block copolymer. Preferred is a random copolymer. According to the present invention, cyclopentene can be suitably recovered even if the ring-opened polymer is a random copolymer.

Examples of the monomer that can co-polymerize with cyclopentene include, but are not particularly limited to, norbornene compounds represented by general formula (1) below. According to the present invention, it is preferred to use a copolymer of cyclopentene and a norbornene compound because cyclopentene can be suitably recovered even if the ring-opened polymer is a copolymer.

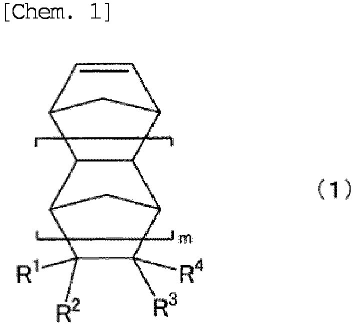

In general formula (1) above, R¹ to R⁴ each represent a hydrogen atom, a hydrocarbon group having 1 to 20 carbon atoms, or a substituent containing a halogen atom, a silicon atom, an oxygen atom, or a nitrogen atom, R² and R³ may be bonded to each other to form a ring, and "m" is an integer of 0 to 2.

Specific examples of the norbornene compound represented by general formula (1) include the following compounds:
bicyclo[2.2.1]hept-2-enes having no substituents or having a hydrocarbon substituent, such as 2-norbornene, 5-methyl-2-norbornene, 5-ethyl-2-norbornene, 5-butyl-2-norbornene, 5-hexyl-2-norbornene, 5-decyl-2-norbornene, 5-cyclohexyl-2-norbornene, 5-cyclopentyl-2-norbornene, 5-ethylidene-2-norbornene, 5-vinyl-2-norbornene, 5-propenyl-2-norbornene, 5-cyclohexenyl-2-norbornene, 5-cyclopentenyl-2-norbornene, 5-phenyl-2-norbornene, tetracyclo [9.2.1.0^{2,10}.0^{3,8}]tetradeca-3,5,7,12-tetraene (also referred to as 1,4-methano-1,4,4a,9a-tetrahydro-9H-fluorene), tetracyclo [10.2.1.0^{2,11}.0^{4,9}]pentadeca-4,6,8,13-tetraene (also referred to as 1,4-methano-1,4,4a,9,9a,10-hexahydroanthracene), dicyclopentadiene, methyl dicyclopentadiene, and dihydrodicyclopentadiene (tricyclo [5.2 .1.0^{2,6}]dec-8-ene) ;
tetracyclo[6.2.1.1^{3,6}.0^{2,7}]dodec-4-enes having no substituents or having a hydrocarbon substituent, such as tetracyclo [6.2.1.1^{3,6}.0^{2,7}] dodec-4-ene, 9-methyltetracyclo [6.2.1.1^{3,6}.0^{2,7}]dodec-4-ene, 9-ethyltetracyclo [6.2 .1.1^{3,6}.0^{2,7}]dodec-4-ene, 9-cyclohexyltetracyclo [6 . 2.1.1^{3,6}.0^{2,7}]dodec-4-ene, 9-cyclopentyltetracyclo [6.2.1.1^{3,6}.0^{2,7}]dodec-4-ene, 9-methylenetetracyclo [6.2.1.1^{3,6}.0^{2,7}]dodec-4-ene, 9-ethylidenetetracyclo [6.2.1.1^{3,6}.0^{2,7}]dodec-4-ene, 9-vinyltetracyclo [6.2.1.1^{3,6}.0^{2,7}]dodec-4-ene, 9-propenyltetracyclo [6.2.1.1^{3,6}.0^{2,7}] dodec-4-ene, 9-cyclohexenyltetracyclo [6.2.1.1^{3,6}.0^{2,7}]dodec-4-ene, 9-cyclopentenyltetracyclo [6.2.1.1^{3,6}.0^{2,7}] dodec-4-ene, and 9-phenyltetracyclo[6.2.1.1^{3,6}.0^{2,7}]dodec-4-ene;
bicyclo[2.2.1]hept-2-enes having an alkoxycarbonyl group, such as methyl 5-norbornene-2-carboxylate, ethyl 5-norbornene-2-carboxylate, methyl 2-methyl-5-norbornene-2-carboxylate, and ethyl 2-methyl-5-norbornene-2-carboxylate;
tetracyclo[6.2.1.1^{3,6}.0^{2,7}]dodec-4-enes having an alkoxycarbonyl group, such as methyl tetracyclo[6.2.1.1^{3,6}.0^{2,7}]dodec-9-ene-4-carboxylate, and methyl 4-methyltetracyclo[6.2.1.1^{3,6}.0^{2,7}] dodec-9-ene-4-carboxylate;
bicyclo[2.2.1]hept-2-enes having a hydroxycarbonyl group or acid anhydride group, such as 5-norbornene-2-carboxylic acid, 5-norbornene-2,3-dicarboxylic acid, and 5-norbornene-2,3-dicarboxylic acid anhydride;
tetracyclo[6.2.1.1^{3,6}.0^{2,7}]dodec-4-enes having a hydroxycarbonyl group or acid anhydride group, such as tetracyclo[6.2.1.1^{3,6}.0^{2,7}]dodec-9-ene-4-carboxylic acid, tetracyclo[6.2.1.1^{3,6}.0^{2,7}]dodec-9-ene-4, 5-dicarboxylic acid, and tetracyclo[6.2.1.1^{3,6}.0^{2,7}]dodec-9-ene-4, 5-dicarboxylic acid anhydride;
bicyclo[2.2.1]hept-2-enes having a hydroxyl group, such as 5-hydroxy-2-norbornene, 5-hydroxymethyl-2-norbornene, 5,6-di(hydroxymethyl)-2-norbornene, 5,5-di(hydroxymethyl)-2-norbornene, 5-(2-hydroxyethoxycarbonyl)-2-norbornene, and 5-methyl-5-(2-hydroxyethoxycarbonyl)-2-norbornene;
tetracyclo [6.2.1.1^{3,6}.0^{2,7}]dodec-4-enes having a hydroxyl group, such as tetracyclo[6.2.1.1^{3,6}.0^{2,7}]dodec-9-ene-4-methanol, and tetracyclo [6.2.1.1^{3,1}.0^{2,7}]dodec-9-en-4-ol;
bicyclo[2.2.1]hept-2-enes having a hydrocarbonyl group, such as 5-norbornene-2-carbaldehyde;
tetracyclo[6.2.1.1^{3,6}.0^{2,7}]dodec-4-enes having a hydrocarbonyl group, such as tetracyclo[6.2.1.1^{3,6}.0^{2,7}]dodec-9-ene-4-carbaldehyde;
bicyclo[2.2.1]hept-2-enes having an alkoxycarbonyl group and a hydroxycarbonyl group, such as 3-methoxycarbonyl-5-norbornene-2-carboxylic acid;
bicyclo[2.2.1]hept-2-enes having a carbonyloxy group, such as 5-norbornene-2-yl acetate, 2-methyl-5-norbornene-2-yl acetate, 5-norbornene-2-yl acrylate, and 5-norbornene-2-yl methacrylate;
tetracyclo[6.2.1.1^{3,6}.0^{2,7}]dodec-4-enes having a carbonyloxy group, such as 9-tetracyclo [6.2.1.1^{3,6}.0^{2,7}]dodec-4-enyl acetate, 9-tetracyclo[6.2.1.1^{3,6}.0^{2,7}] dodec-4-enyl acetate, and 9-tetracyclo[6.2.1.1^{3,6}.0^{2,7}] dodec-4-enyl methacrylate;
bicyclo[2.2.1]hept-2-enes having a nitrogen-containing functional group, such as 5-norbornene-2-carbonitrile, 5-norbornene-2-carboxyamide, and 5-norbornene-2,3-dicarboxic acid imide;
tetracyclo[6.2.1.1^{3,6}.0^{2,7}]dodec-4-enes having a nitrogen-containing functional group, such as tetracyclo[6.2.1.1^{3,6}.0^{2,7}]dodec-9-ene-4-carbonitrile, tetracyclo [6.2.1.1^{3,6}.0^{2,7}] dodec-9-ene-4-carboxyamide, and tetracyclo[6.2.1.1^{3,6}.0^{2,7}] dodec-9-ene-4, 5-dicarboxic acid imide;
bicyclo[2.2.1]hept-2-enes having a halogen atom, such as 5-chloro-2-norbornene;
tetracyclo[6.2.1.1^{3,6}.0^{2,7}]dodec-4-enes having a halogen atom, such as 9-chlorotetracyclo[6.2.1.1^{3,6}.0^{2,7}]dodec-4-ene;
bicyclo[2.2.1]hept-2-enes having a silicon atom-containing functional group, such as 5-trimethoxysilyl-2-norbornene and 5-triethoxysilyl-2-norbornene;
tetracyclo[6.2.1.1^{3,6}.0^{2,7}]dodec-4-enes having a silicon atom-containing functional group, such as 4-trimethoxysilyltetracyclo[6.2.1.1^{3,6}.0^{2,7}]dodec-9-ene and 4-triethoxysilyltetracyclo[6.2.1.1^{3,6}.0^{2,77}]dodec-9-ene.

As the above norbornene compounds represented by general formula (1), compounds where "m" in general formula (1) is 0 or 1 are preferred. In general formula (1), R¹ to R⁴ may be the same or different.

Examples of the monomer that can co-polymerize with cyclopentene also include monocyclic olefins other than cyclopentene, polycyclic cycloolefins having an aromatic ring, and the like. The monocyclic olefins other than cyclopentene may be any olefin having only one cyclic structure. Examples thereof include, but are not limited to, cyclic monoolefins such as cyclopropene, cyclobutene, methylcyclopentene, cyclohexene, methylcyclohexene, cycloheptene, and cyclooctene; cyclic diolefins such as cyclohexadiene, methylcyclohexadiene, cyclooctadiene, and methylcyclooctadiene; and the like. Examples of the polycyclic cycloolefins having an aromatic ring include phenylcyclooctene, 5-phenyl-1,5-cyclooctadiene, phenylcyclopentene, and the like.

From the viewpoint of recovering efficiency, the ring-opened polymer used in the present invention is preferably a cyclopentene homopolymer or a copolymer of cyclopentene and a norbornene compound. The content of cyclopentene-derived structural units in the ring-opened polymer used in the present invention is preferably 5 to 100% by weight, more preferably 10 to 100% by weight, still more preferably 15 to 100% by weight based on the total amount of the structural units as 100% by weight, but not particularly limited thereto.

The weight average molecular weight (Mw) of the ring-opened polymer used in the present invention is preferably 10,000 to 2,000,000, more preferably 20,000 to 1,000,000, still more preferably 30,000 to 750,000 as a weight average molecular weight (Mw) measured against polystyrene standards by gel permeation chromatography. The ratio (Mw/Mn) between the weight average molecular weight (Mw) and the number average molecular weight (Mn) of the ring-opened polymer used in the present invention measured against polystyrene standards by gel permeation chromatography is preferably 1.0 to 5.0, more preferably 1.5 to 3.0.

The ring-opened polymer used in the present invention has a glass transition temperature (Tg) of preferably -110 to 10°C, more preferably -105 to 5°C, still more preferably -100 to 0°C. The glass transition temperature of the ring-opened polymer can be controlled by selecting the type and the amount of the monomer used, for example.

The ring-opened polymer used in the present invention may have modifying groups at polymer chain ends. Although not particularly limited, the modifying groups introduced to polymer chain ends are preferably modifying groups containing an atom selected from the group consisting of atoms of the Group 15 elements in the periodic table, atoms of the Group 16 elements in the periodic table, and a silicon atom.

Examples of modifying groups containing a nitrogen atom include amino, pyridyl, imino, amido, nitro, and urethane bond groups, and hydrocarbon groups containing these groups. Examples of modifying groups containing an oxygen atom include hydroxyl, carboxylic acid, ether, ester, carbonyl, aldehyde, and epoxy groups, and hydrocarbon groups containing these groups. Examples of modifying groups containing a silicon atom include alkylsilyl and oxysilyl groups, and hydrocarbon groups containing these groups. Examples of modifying groups containing a phosphorus atom include phosphoric acid and phosphino groups, and hydrocarbon groups containing these groups. Examples of modifying groups containing a sulfur atom include sulfonyl, thiol, and thioether groups, and hydrocarbon groups containing these groups. Alternatively, modifying groups containing two or more of the above groups may be used.

The method for producing the ring-opened polymer used in the present invention is not particularly limited, and examples thereof include a method in which ring-opening polymerization of monomers containing cyclopentene is performed in a solvent in the presence of a ring-opening polymerization catalyst. Examples of the ring-opening polymerization catalyst include a combination of a transition metal of Group 6 of the periodic table as a major catalyst and an organometallic compound as a catalytic promoter, a metathesis catalyst such as a ruthenium-carbene complex, and the like. In the method for producing the ring-opened polymer used in the present invention, an olefin compound or a diolefin compound may be added as a molecular weight modifier to the polymerization system.

The ring-opened polymer used in the present invention may be a cross-linked polymer (a cross-linked rubber). The cross-linked polymer may be those obtained by adding necessary amounts of compounding agents such as a cross-linking agent, a cross-linking accelerator, a cross-linking activator, a process oil, a filler, an antioxidant, an activator, a plasticizer, and a lubricant to the above-described ring-opened polymer to prepare a polymer composition, and crosslinking the resulting polymer composition. Such a cross-linked polymer contains a cross-linked ring-opened polymer and the above-described compounding agents, and can be suitably used in tire applications, for example, in tire parts such as treads, carcasses, sidewalls, beads, and the like. When the ring-opened polymer used in the present invention is a cross-linked polymer, the above-described compounding agents may not be contained.

Examples of the cross-linking agent include sulfurs such as powdered sulfur, precipitated sulfur, colloidal sulfur, insoluble sulfur, and highly dispersible sulfur; halogenated sulfurs such as sulfur monochloride and sulfur dichloride; organic peroxides such as dicumyl peroxide and ditertiary butyl peroxide; quinone dioximes such as p-quinone dioxime and p,p'-dibenzoylquinone dioxime; organic polyvalent amine compounds such as triethylenetetramine, hexamethylenediamine carbamate, and 4,4'-methylene bis-o-chloroaniline; alkylphenol resins having a methylol group; and the like.

Examples of the cross-linking accelerator include sulfenamide-based cross-linking accelerators, guanidine-based cross-linking accelerators, thiourea-based cross-linking accelerators, thiazole-based cross-linking accelerators, thiuram-based cross-linking accelerators, dithiocarbamic acid-based cross-linking accelerators, xanthogenic acid-based cross-linking accelerators; and the like.

Examples of the filler include carbon black and silica.

The cross-linked polymer used in the present invention may contain a rubber other than the above-described ring-opened polymer having cyclopentene-derived structural units as a rubber component, and examples of such a rubber include natural rubber (NR), polybutadiene rubber (BR), polyisoprene rubber (IR), styrene-butadiene copolymer rubber (SBR), and the like.

### <Method for recovering cyclopentene>

The method for recovering cyclopentene according to the present invention is a method for recovering cyclopentene from the above-described ring-opened polymer having a structural unit derived from cyclopentene by allowing a metathesis catalyst to act on the above-described ring-opened polymer having a structural unit derived from cyclopentene.

In the method for recovering cyclopentene according to the present invention, cyclopentene is recovered from the cyclopentene-derived structural units represented by Formula (2) below by the action of a metathesis catalyst on cyclopentene-derived structural units represented by Formula (2) below contained in a ring-opened polymer.

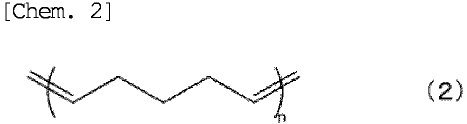

Specifically, according to the present invention, depolymerization of cyclopentene-derived structural units by a metathesis catalyst can proceed as shown in Formulae (I) to (VI), and thus cyclopentene can be recovered. In the reactions in Formulae (I) to (VI), after the reaction shown in Formula (VI) is completed, the reaction shown in Formula (IV) below occurs. Thus, the reactions shown in Formulae (IV), (V), and (VI) occur successively, and thus cyclopentene can be recovered successively. In Formulae (I) to (VI), an exemplary embodiment using a ruthenium-carbene compound is shown, but the present invention is not particularly limited to the embodiment using a ruthenium-carbene compound.

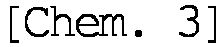

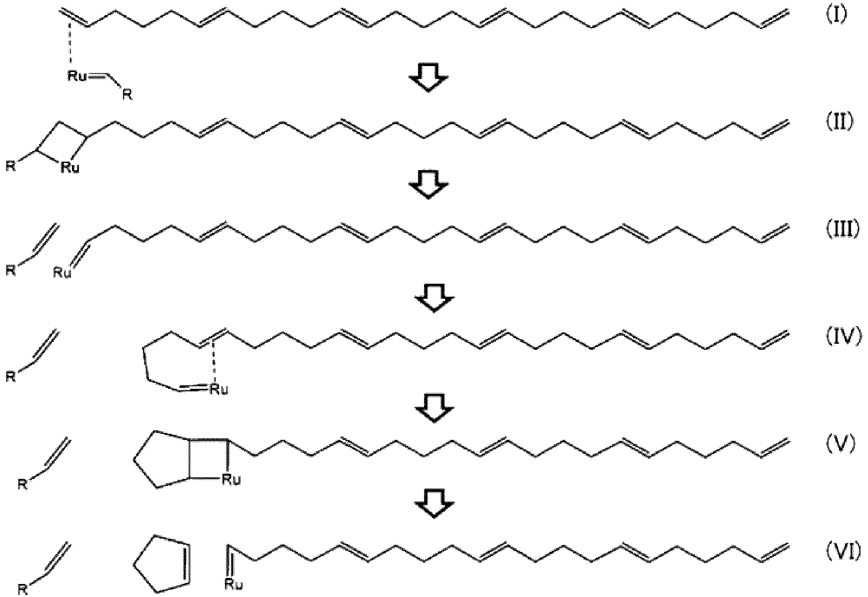

When the ring-opened polymer is a cyclopentene homopolymer, the ring-opened polymer substantially consists of only cyclopentene, and therefore the purity of cyclopentene in the monomers produced by depolymerization can be high.

When the ring-opened polymer is a copolymer of cyclopentene and a monomer that can co-polymerize with cyclopentene, the depolymerization of the cyclopentene-derived structural units by a metathesis catalyst primarily proceeds in the cyclopentene-derived structural units. Thus, depolymerization may or may not proceed in the structural units derived from the co-polymerizable monomers, depending on the monomers constituting the structural units. For example, when the ring-opened polymer is a copolymer of cyclopentene and a norbornene compound, depolymerization by a metathesis catalyst primarily proceeds in the cyclopentene-derived structural units, and depolymerization in the structural units derived from the norbornene compound by a metathesis catalyst hardly proceeds due to the structure thereof. Since depolymerization in the structural units derived from the norbornene compound hardly proceeds, the purity of cyclopentene in the monomers obtained by the depolymerization can also be high in this case.

Furthermore, when the ring-opened polymer is a cross-linked polymer, among cyclopentene-derived structural units contained in the ring-opened polymer, depolymerization by a metathesis catalyst can proceed in the cyclopentene-derived structural units except for those constituting crosslinking points, and thus cyclopentene can be recovered.

The metathesis catalyst used in the present invention is a complex in which a plurality of ions, atoms, polyatomic ions, and/or compounds are bonded to a transition metal atom as a central atom. As the transition metal atom, atoms of Groups 5, 6 and 8 of the periodic table (the short form of periodic table, the same applies hereinafter) are used. Examples of the transition metal atom include, but are not particularly limited to, vanadium, niobium, and tantalum as atoms of Group 5; molybdenum and tungsten as atoms of Group 6; and ruthenium and osmium as atoms of Group 8.

Among these, preferably used as a metathesis catalyst are complexes of molybdenum or tungsten of Group 6 and ruthenium or osmium of Group 8, and particularly preferred are complex catalysts of molybdenum or tungsten and carbene complexes of molybdenum, tungsten, or ruthenium. A ruthenium-carbene complex is preferred because it is relatively stable toward oxygen or moisture in the air, hardly inactivated, and excellent in catalytic activity.

More specifically, examples of the complex catalyst containing a transition metal of Group 6 of the periodic table include tungsten hexachloride, tungsten oxytetrachloride, tungsten(ethylimide)(tetrachloride)(diethyl ether), tungsten (ethylimide) (tetrachloride) (tetrahydrofuran), tungsten (phenylimide) (tetrachloride) (diethyl ether), tungsten(phenylimide)(tetrachloride)(tetrahydrofuran), tungsten(2,6-dimethylphenylimide) (tetrachloride)(diethyl ether), bis{3,3'-di(t-butyl)-5,5',6,6'-tetramethyl-2,2'-biphenoxy}oxymolybdenum(VI), bis{3,3',5,5'-tetramethyl-2,2'-biphenoxy}oxymolybdenum(VI), bis{3,3'-di(t-butyl)-5,5',6,6'-tetramethyl-2,2'-biphenoxy}oxytungsten(VI), {3,3'-di(t-butyl)-5,5',6,6'-tetramethyl-2,2'-biphenoxy}oxytungsten(VI) dichloride, and the like.

When the above-described complex catalyst containing a transition metal of Group 6 of the periodic table is used as a metathesis catalyst, in order to increase catalytic activity, it is preferred to use the above-described complex catalyst containing a transition metal of Group 6 of the periodic table in combination with a different catalyst than the complex catalyst as a complex catalyst.

Examples of the different catalyst include known organometallic compounds. As the organometallic compounds, preferred is an organometallic compound of any one of Groups 1, 2, 12, 13, and 14 of the periodic table having a hydrocarbon group having 1 to 20 carbon atoms, more preferred are organolithium compounds, organomagnesium compounds, organozinc compounds, organoaluminum compounds, and organotin compounds, and particularly preferred are organolithium compounds and organoaluminum compounds.

Examples of the organolithium compounds include n-butyllithium, methyllithium, phenyllithium, neopentyllithium, neophyllithium, and the like.

Examples of the organomagnesium compounds include butylethylmagnesium, butyloctylmagnesium, dihexylmagnesium, ethylmagnesium chloride, n-butylmagnesium chloride, allylmagnesium bromide, neopentylmagnesium chloride, neophylmagnesium chloride, and the like.

Examples of the organozinc compounds include dimethylzinc, diethylzinc, diphenylzinc, and the like.

Examples of the organoaluminum compounds include trimethylaluminum, triethylaluminum, triisobutylaluminum, diethylaluminum chloride, diethylaluminum ethoxide, ethylaluminum dichloride, ethylaluminum diethoxide, diisobutylaluminium mono(n-hexoxide), and the like.

Examples of the organotin compounds include tetramethyltin, tetra(n-butyl)tin, tetraphenyltin, and the like.

These different catalysts may be used alone or in combination.

In addition, examples of the complex catalyst containing a transition metal of Group 6 of the periodic table include compounds represented by general formula (3) below.

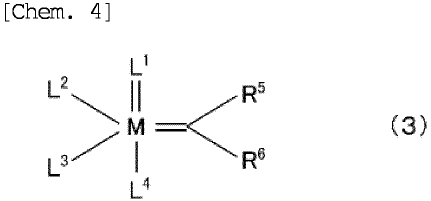

In general formula (3), M represents a molybdenum atom or a tungsten atom.

R⁵ and R⁶ each independently represent a hydrogen atom; an alkyl group having 1 to 12 carbon atoms, such as a methyl group, an ethyl group, an n-propyl group, an isopropyl group, an n-butyl group, an isobutyl group, or a t-butyl group; a cycloalkyl group having 3 to 20 carbon atoms, such as a cyclopropyl group, a cyclopentyl group, or a cyclohexyl group; or an optionally substituted aryl group having 6 to 20 carbon atoms. Examples of the aryl group of the optionally substituted aryl group include a phenyl group, a 1-naphthyl group, a 2-naphthyl group, and the like. Examples of the substituent of the aryl group include alkyl groups having 1 to 12 carbon atoms, such as a methyl group and an ethyl group; halogen atoms such as a fluorine atom, a chlorine atom, and a bromine atom; alkoxy groups having 1 to 12 carbon atoms, such as a methoxy group, an ethoxy group, and an isopropoxy group; haloalkyl groups having 1 to 12 carbon atoms, such as a trifluoromethyl group; haloalkoxy groups having 1 to 12 carbon atoms, such as a trifluoromethoxy group; optionally substituted aryl groups having 6 to 12 carbon atoms, such as a phenyl group, a 4-methylphenyl group, a 2,4-dimethylphenyl group, a 2-chlorophenyl group, and a 3-methoxyphenyl group; and the like.

L¹ represents a nitrogen atom or an oxygen atom optionally having a substituent selected from an alkyl group having 1 to 12 carbon atoms, an optionally substituted aryl group having 6 to 20 carbon atoms, and an optionally substituted cycloalkyl group having 3 to 20 carbon atoms.

The alkyl group having 1 to 12 carbon atoms as a substituent of the nitrogen atom or the oxygen atom as L¹ may be a linear, branched, or cyclic group. Specific examples thereof include a methyl group, an ethyl group, a propyl group, an isopropyl group, a butyl group, a t-butyl group, a pentyl group, a hexyl group, and the like.

Examples of the cycloalkyl group having 3 to 20 carbon atoms as a substituent of the nitrogen atom or the oxygen atom as L¹ include a cyclopropyl group, a cyclopentyl group, a cyclohexyl group, a cyclooctyl group, an adamantyl group, and the like.

Examples of the aryl group having 6 to 12 carbon atoms as a substituent of the nitrogen atom or the oxygen atom as L¹ include a phenyl group, a 1-naphthyl group, a 2-naphthyl group, and the like.

The substituent as an optional substituent of the cycloalkyl group having 3 to 20 carbon atoms or the aryl group having 6 to 12 carbon atoms of the nitrogen atom or the oxygen atom as L¹ is not particularly limited. Examples of the substituent include alkyl groups having 1 to 12 carbon atoms, such as a methyl group and an ethyl group; halogen atoms such as a fluorine atom, a chlorine atom, and a bromine atom; alkoxy groups having 1 to 12 carbon atoms, such as a methoxy group, an ethoxy group, and an isopropoxy group; haloalkyl groups having 1 to 12 carbon atoms, such as a trifluoromethyl group; haloalkoxy groups having 1 to 12 carbon atoms, such as a trifluoromethoxy group; optionally substituted aryl groups having 6 to 12 carbon atoms, such as a phenyl group, a 4-methylphenyl group, a 2,4-dimethylphenyl group, a 2-chlorophenyl group, and a 3-methoxyphenyl group; an amino group; monosubstituted amino groups such as a methylamino group; disubstituted amino groups such as a dimethylamino group; an imino group; and the like.

L² and L³ are each an optionally substituted 5- to 15-membered conjugated heterocyclic group having at least one nitrogen atom or an alkoxy group including O-R⁷ where R⁷ is a group selected from an optionally substituted alkyl group having 1 to 12 carbon atoms and an optionally substituted aryl group having 6 to 30 carbon atoms.

Examples of the conjugated heterocyclic group as L² and L³ include 5-membered conjugated heterocyclic groups such as a pyrrolyl group, an imidazolyl group, a pyrazolyl group, an oxazolyl group, and a thiazolyl group; 6-membered conjugated heterocyclic groups such as a pyridyl group, a pyridazinyl group, a pyrimidinyl group, a pyrazinyl group, and a triazinyl group; fused-ring conjugated heterocyclic groups such as a quinazolinyl group, a phthalazinyl group, and a pyrrolopyridyl group; and the like.

The substituent as an optional substituent of the conjugated heterocyclic group is not particularly limited. Examples of the substituent include alkyl groups having 1 to 12 carbon atoms, such as a methyl group and an ethyl group; halogen atoms such as a fluorine atom, a chlorine atom, and a bromine atom; alkoxy groups having 1 to 12 carbon atoms, such as a methoxy group, an ethoxy group, and an isopropoxy group; haloalkyl groups having 1 to 12 carbon atoms, such as a trifluoromethyl group; haloalkoxy groups having 1 to 12 carbon atoms, such as a trifluoromethoxy group; optionally substituted aryl groups having 6 to 12 carbon atoms, such as a phenyl group, a 4-methylphenyl group, a 2,4-dimethylphenyl group, a 2-chlorophenyl group, and a 3-methoxyphenyl group; an amino group; monosubstituted amino groups such as a methylamino group; disubstituted amino groups such as a dimethylamino group; an imino group; and the like.

Examples of the alkyl group having 1 to 12 carbon atoms of the optionally substituted alkyl group having 1 to 12 carbon atoms as R⁷ include a methyl group, an ethyl group, a propyl group, an isopropyl group, a butyl group, a t-butyl group, and a pentyl group. When both L² and L³ include O-R⁷, the alkyl groups of R⁷ may be bonded to each other.

The substituent as an optional substituent of the alkyl group having 1 to 12 carbon atoms as R⁷ is not particularly limited. Examples of the substituent include halogen atoms such as a fluorine atom, a chlorine atom, and a bromine atom; alkoxy groups having 1 to 12 carbon atoms, such as a methoxy group, an ethoxy group, and an isopropoxy group; haloalkyl groups having 1 to 12 carbon atoms, such as a trifluoromethyl group; haloalkoxy groups having 1 to 12 carbon atoms, such as a trifluoromethoxy group; optionally substituted aryl groups having 6 to 12 carbon atoms, such as a phenyl group, a 4-methylphenyl group, a 2,4-dimethylphenyl group, a 2-chlorophenyl group, and a 3-methoxyphenyl group; an amino group; monosubstituted amino groups such as a methylamino group; disubstituted amino groups such as a dimethylamino group; an imino group; and the like.

Specific examples of R⁷ include a 1,1,1,3,3,3-hexafluoro-2-propoxy group, a 2-methyl-2-propoxy group, a 1,1,1-trifluoro-2-methyl-2-propoxy group, a 1,1,1-trifluoro-2-trifluoromethyl-2-propoxy group, and a 2-trifluoromethyl-2-phenyl-1,1, 1-trifluoroethoxy group. Specific examples of the optionally substituted aryl group having 6 to 30 carbon atoms include a 2,6-bis(2,4,6-trimethylphenyl)phenoxy group, a 2,6-bis(2,4,6-triisopropylphenyl)phenoxy group, a 2,4,6-trimethylphenoxy group, a 2,3,5,6-tetraphenylphenoxy group, and the like. Examples of such groups in which the alkyl groups of R⁷ are bonded to each other include a 3,3'-di(t-butyl)-5,5',6,6'-tetramethyl-2,2'-biphenoxy group.

L⁴ is a neutral conjugated heterocyclic ligand having at least one nitrogen atom, that is, a 12- to 24-membered conjugated heterocyclic group. Specific examples of the neutral conjugated heterocyclic ligand having a nitrogen atom include pyridine, 2-methylpyridine, 2,4-lutidine, 2,6-lutidine, 2,2'-bipyridine, 5,5'-dimethyl-2,2'-bipyridyl, 4,4'-dimethyl-2,2'-bipyridyl, 4,4'-dibromo-2,2'-bipyridyl, 2,2'-biquinoline, 1,10-phenanthroline, and terpyridine. When a plurality of nitrogen atoms are included, the ligand can be a bidentate ligand rather than a monodentate ligand.

The conjugated heterocyclic group of L⁴ may have a substituent. Examples of the substituent include the same substituents as those enumerated above as the optional substituents of the conjugated heterocyclic group as L².

Specific examples of the compounds represented by general formula (3) include, but are not limited to, bis(1,1,1,3,3,3-hexafluoro-2-propoxy)neophylidenetungsten(VI) (2,6-dimethylphenylimido), bis(1,1,1,3,3,3-hexafluoro-2-propoxy)neophylidenemolybdenum (VI) (2,6-dimethylphenylimido), bis(1,1,1,3,3,3-hexafluoro-2-propoxy)neophylidenetungsten(VI) (2,6-diisopropylphenylimido), (2,3,5,6-tetraphenylphenoxy)2,6-dimethylphenylimidetungsten(VI) (2,5-dimethylpyrrolide)(neophylidene), {3,3'-di(t-butyl)-5,5',6,6'-tetramethyl-2,2'-biphenoxy}neophylidenemolybdenum(VI) (2,6-dimethylphenylimido), (2-trifluoromethyl-2-phenyl-1,1,1-trifluoroethoxy)2,6-dimethylphenylimidetungsten(VI) (2,5-dimethylpyrrolide)(neophylidene)(1,10-phenanthroline), (2-trifluoromethyl-2-phenyl-1,1,1-trifluoroethoxy)2,6-dimethylphenylimidetungsten(VI) (2,5-dimethylpyrrolide)(neophylidene)(2,2'-bipyridine), (2-trifluoromethyl-2-phenyl-1,1,1-trifluoroethoxy)phenylimidotungsten(VI)(2,5-dimethylpyrrolide)(neophylidene)(pyridine), and the like.

As a ruthenium-carbene complex, complex compounds represented by general formula (4) or general formula (5) below are preferred.

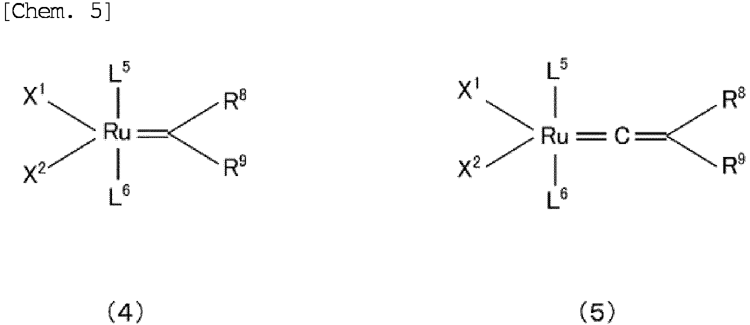

In general formulae (4) and (5) above, R⁸ and R⁹ are each independently a hydrogen atom; a halogen atom; or a hydrocarbon group having 1 to 20 carbon atoms optionally containing a halogen atom, an oxygen atom, a nitrogen atom, a sulfur atom, a phosphorus atom, or a silicon atom. X¹ and X² are each independently any anionic ligand, and L⁵ and L⁶ are each independently a heteroatom-containing carbene compound or a neutral electron-donating compound. R⁸, R⁹, X¹, X², L⁵, and L⁶ may be bonded to each other in any combination to form a multidentate chelating ligand. A "hetero atom" refers to an atom of Groups 15 and 16 of the periodic table, and specific examples thereof include N, O, P, S, As, and Se atoms, and the like. Among these, N, O, P, and S atoms are preferred, and an N atom is particularly preferred because a stable carbene compound can be obtained.

As a heteroatom-containing carbene compound, preferred is a carbene compound having heteroatoms adjacently bonded to both sides of the carbene carbon, and more preferred is a carbene compound having a heterocycle constituted by the carbene carbon atom and two heteroatoms present at both sides thereof. The heteroatoms adjacent to the carbene carbon preferably have a bulky substituent. As the heteroatom-containing carbene compound, compounds represented by general formula (6) or (7) below are preferred.

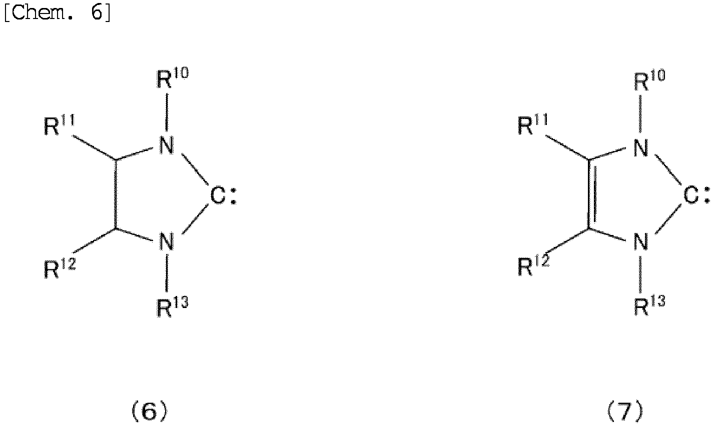

In general formulae (6) and (7) above, R¹⁰ to R¹³ are each independently a hydrogen atom; a halogen atom; or a hydrocarbon group having 1 to 20 carbon atoms optionally containing a halogen atom, an oxygen atom, a nitrogen atom, a sulfur atom, a phosphorus atom, or a silicon atom. R¹⁰ to R¹³ may be bonded to each other in any combination to form a multidentate chelating ligand.

In general formulae (4) and (5) above, anionic ligands X¹ and X² are ligands each having a negative charge when liberated from the central metal, and examples thereof include halogen atoms such as F, Cl, BR, and I; a diketonate group, substituted cyclopentadienyl groups, alkoxy groups, aryloxy groups, and a carboxyl group. Among these, halogen atoms are preferred, and a chlorine atom is more preferred.

The neutral electron-donating compound may be any ligand that has a neutral charge when liberated from the central metal, and examples thereof include carbonyls, amines, pyridines, ethers, nitriles, esters, phosphines, thioethers, aromatic compounds, olefins, isocyanides, thiocyanates, and the like. Among these, phosphines, ethers, and pyridines are preferred, and trialkylphosphine is more preferred.

Specific examples of the complex compound represented by general formulae (4) and (5) above include ruthenium complex compounds in which L⁵ and L⁶ are a heteroatom-containing carbene compound and a neutral electron-donating compound, respectively, such as benzylidene (1,3-dimesitylimidazolidin-2-ylidene)(tricyclohexylphosphine)ruthenium dichloride, (1,3-dimesitylimidazolidin-2-ylidene) (3-methyl-2-butene-1-ylidene)(tricyclopentylphosphine)ruthenium dichloride, benzylidene(1,3-dimesityl-2,3-dihydrobenzimidazol-2-ylidene) (tricyclohexylphosphine) ruthenium dichloride, and tricyclohexylphosphine[1,3-bis(2,4,6-trimethylphenyl)-4,5-dihydroimidazol-2-ylidene][3-methyl-2-butenylidene]ruthenium dichloride; ruthenium compounds in which L⁵ and L⁶ are both a neutral electron-donating compound, such as benzylidenebis(tricyclohexylphosphine)ruthenium dichloride, dichloro-(3-phenyl-1H-inden-1-ylidene)bis(tricyclohexylphosphine)ruthenium, and (3-methyl-2-buten-1-ylidene)bis(tricyclopentylphosphine)ruthenium dichloride; ruthenium complex compounds in which L⁵ and L⁶ are both a heteroatom-containing carbene compound, such as benzylidenebis(1,3-dicyclohexylimidazolidin-2-ylidene)ruthenium dichloride and benzylidenebis(1,3-diisopropyl-4-imidazolin-2-ylidene)ruthenium dichloride; and the like. Among these, preferred are ruthenium complex compounds in which L⁵ and L⁶ are a heteroatom-containing carbene compound and a neutral electron-donating compound, respectively.

Specific examples of the compounds represented by general formula (6) or (7) above include 1,3-dimethylimidazolidin-2-ylidene, 1,3-dimesityl-4-imidazolin-2-ylidene, 1,3-di(1-phenylethyl)-4-imidazolin-2-ylidene, and 1,3-dimesityl-2,3-dihydrobenzimidazol-2-ylidene. In addition to the compounds represented by general formulae (6) and (7) above, for example, a heteroatom-containing carbene complex compound such as 1,3,4-triphenyl-2,3,4,5-tetrahydro-1H-1,2,4-triazol-5-ylidene or 1,3,4-triphenyl-4,5-dihydro-1H-1,2,4-triazol-5-ylidene can also be used.

These metathesis catalysts may be used alone or in combination.

In the recovery method according to the present invention, the amount of the metathesis catalyst used is preferably 0.001 to 15 parts by weight, more preferably 0.001 to 5 parts by weight, still more preferably 0.005 to 4 parts by weight, even more preferably 0.01 to 3 parts by weight with respect to 100 parts by weight of the ring-opened polymer at the beginning of the reaction. When the amount of the metathesis catalyst used is within the above range, cyclopentene can be more efficiently recovered. When a cross-linked polymer is used as the ring-opened polymer, the amount of the metathesis catalyst used is more preferably in the range of 0.001 to 10 parts by weight, still more preferably in the range of 0.01 to 7.5 parts by weight with respect to 100 parts by weight of the polymer component at the beginning of the reaction.

In the recovery method according to the present invention, the depolymerization reaction is preferably carried out in a solvent in a state where a ring-opened polymer and a metathesis catalyst are dispersed or dissolved, and the depolymerization reaction may be performed with stirring. In this case, from the viewpoint of suppressing re-polymerization of the recovered cyclopentene by the action of the metathesis catalyst and thus allowing the depolymerization reaction to suitably proceed, the depolymerization reaction is preferably performed such that the amount of cyclopentene produced by depolymerization in a solvent is relatively low. Specifically, cyclopentene is preferably recovered by allowing a metathesis catalyst to act on a ring-opened polymer in a solvent while controlling the concentration of cyclopentene to be in a range of 1.7 mol/L or less. Further, cyclopentene is recovered by allowing a metathesis catalyst to act on a ring-opened polymer while controlling the concentration of cyclopentene in a solvent to be more preferably 1.60 mol/L or less, still more preferably 1.30 mol/L or less. Examples of the method for controlling the concentration of cyclopentene in a solvent to be in the above-described range when cyclopentene is recovered include, but are not particularly limited, a method for recovering cyclopentene while controlling the amount of a ring-opened polymer in a solvent to be relatively low, a method for allowing depolymerization of a ring-opened polymer to proceed while recovering/removing cyclopentene produced by the depolymerization from the solvent, and the like. The cyclopentene produced by depolymerization can be recovered/removed from the solvent, for example, by a volatilization method. The lower limit of the concentration of cyclopentene is preferably, but not limited to, 0.01 mol/L or more (however, since cyclopentene is not substantially present immediately after the beginning of the reaction, the concentration of cyclopentene may be less than 0.01 mol/L).

Instead of using the method for controlling the concentration of cyclopentene in a solvent to be in the above-described range when cyclopentene is recovered, the recovery amount of cyclopentene obtained in a predetermined time can be increased and the cyclopentene recovery rate can also be increased by suitably reducing the concentration of cyclopentene at the beginning of a reaction based on the assumption that the reaction completely occurs and cyclopentene is all recovered, that is, the concentration of polycyclopentene repeating units at the beginning of the reaction. Specifically, cyclopentene is preferably recovered by allowing a metathesis catalyst to act on a ring-opened polymer while controlling the amount of the ring-opened polymer used at the beginning of the reaction such that the concentration of polycyclopentene repeating units in the ring-opened polymer is 5 mol/L or less. Further, cyclopentene is preferably recovered by allowing a metathesis catalyst to act on a ring-opened polymer while controlling the amount of the ring-opened polymer used at the beginning of the reaction such that the concentration of polycyclopentene repeating units in the ring-opened polymer is more preferably 4.0 mol/L or less, and still more preferably 3.0 mol/L or less. The lower limit of the amount of a ring-opened polymer used at the beginning of the reaction is preferably, but not limited to, such an amount that the concentration of polycyclopentene repeating units in the ring-opened polymer is 0.1 mol/L or more.

The solvent may be any solvent that can disperse or dissolve a ring-opened polymer and a metathesis catalyst, and is not particularly limited. Examples thereof include aromatic hydrocarbons such as benzene, toluene, xylene, and ethylbenzene; aliphatic hydrocarbons such as hexane, n-heptane, and n-octane; alicyclic hydrocarbons such as cyclohexane, cyclopentane, and methylcyclohexane; haloalkanes such as dichloromethane, chloroform; aromatic halogens such as chlorobenzene and dichlorobenzene; and the like.

When the depolymerization reaction is allowed to proceed in a solvent in a state where a ring-opened polymer and a metathesis catalyst are dispersed or dissolved, the reaction temperature is preferably 0 to 100°C, more preferably 10 to 80°C, still more preferably 20 to 70°C, and the reaction time is preferably 1 minute to 100 hours, more preferably 10 minutes to 24 hours.

In addition, optionally, a reaction-terminating agent is added to the reaction system to terminate the depolymerization reaction, and thereby the recovered cyclopentene can be obtained as a cyclopentene solution in which the cyclopentene is dispersed or dissolved in the solvent. The cyclopentene solution thus obtained contains a reaction residue of the metathesis catalyst. Thus, according to the recovery method according to the present invention, a cyclopentene solution in which cyclopentene and a reaction residue of a metathesis catalyst is contained in a solvent can be obtained.

In the present invention, from the viewpoint of suppressing re-polymerization of the recovered cyclopentene by the action of the metathesis catalyst and thus allowing the depolymerization reaction to suitably proceed, the recovery of cyclopentene is desirably performed in a state where the amount of cyclopentene produced by depolymerization in a solvent is relatively low, and thus the cyclopentene solution obtained has a low concentration of cyclopentene at 25°C, which is preferably 0.01 to 1.7 mol/L. The concentration of cyclopentene in a cyclopentene solution obtained by the recovery method according to the present invention is preferably 0.05 to 1.65 mol/L, more preferably 0.1 to 1.6 mol/L.

Reaction residues of the metathesis catalyst contained in the cyclopentene solution are not particularly limited. For example, when a copolymer of cyclopentene and a monomer that can co-polymerize with cyclopentene is used as a ring-opened polymer, the depolymerization reaction of structural structures other than cyclopentene or the depolymerization reaction of part of cyclopentene structural units may not proceed in some cases. In such cases, a product produced by binding of a group derived from the metathesis catalyst to the end of a polymer chain that has not undergone depolymerization is contained as a reaction residue of the metathesis catalyst.

When a cross-linked polymer is used as a ring-opened polymer, more specifically, when a cross-linked polymer containing a filler such as carbon black or silica is used, in addition to the recovered cyclopentene and a reaction residue of the metathesis catalyst, the filler such as carbon black or silica is dispersed in the cyclopentene solution after depolymerization.

In the recovery method according to the present invention, cyclopentene can be isolated from the thus-obtained cyclopentene solution containing cyclopentene and a reaction residue of the metathesis catalyst by a conventional method such as filtration, distillation, extraction, extractive distillation, absorption, or the like.

### EXAMPLES

Hereinafter, the present invention is more specifically described by way of Examples and Comparative Examples, but the present invention is not limited to the examples. The term "part(s)" refers to part(s) by weight unless otherwise indicated. Test methods used in Examples and Comparative Examples are as follows.

### <Molecular weight>

The number average molecular weight (Mn), the weight average molecular weight (Mw), and the molecular weight distribution (Mw/Mn) of each ring-opened polymer were determined as values measured against polystyrene standards by gel permeation chromatography (GPC) using tetrahydrofuran as a solvent. A measurement system "HLC-8320GPC" (available from Tosoh Corporation) was used. The measurement was performed using four "TSKgel SuperMultiporeHZ-H" as columns connected in series under the following conditions: flow rate: 0.35 ml/min, sample injection volume: 10 µml, and column temperature: 40°C.

### <Contents of cyclopentene structural units and norbornene compound structural units>

The ratios of monomer units in the ring-opened polymer were determined by ¹H-NMR spectrometry. Specifically, ¹H-NMR measurement was performed using deuteriochloroform as a solvent, and the ratios of the monomer units in the ring-opened polymer were determined based on the ratios of signal integrals in 5.0 to 5.5 ppm derived from double bonds and the ratios of signal integrals in 2.3 to 3.0 ppm derived from norbornene compounds.

### <Glass transition temperature (Tg)>

The glass transition temperature (Tg) of each ring-opened polymer was measured with a differential scanning calorimeter (DSC) at a temperature increase rate of 10°C/min.

### <Amount of recovered cyclopentene>

The solution after depolymerization was measured by gas chromatography (GC) to calculate the amount of recovered cyclopentene. Specifically, a measurement system "GC-2025" (available from Tosoh Corporation) was used. The measurement was performed using "InterCap 1" (available from GL Sciences Inc.) as a column under the following conditions: N₂ gas flow rate: 1.2 ml/min, sample injection volume: 10 pml, and increase in column temperature: from 35°C to 280°C.

### <Amount of cyclopentene in polymer or cross-linked rubber used for recovery>

In the reaction mixture before depolymerization, the content of segments derived from polycyclopentene contained in the system was calculated in parts by mol based on the contents of cyclopentene structural units and norbornene compound structural units obtained by ¹H-NMR spectrometry. Further, the content thus obtained was multiplied by the molecular weight to obtain the amount of cyclopentene in the polymer or the cross-linked rubber used for the recovery.

### <Cyclopentene recovery rate>

The cyclopentene recovery rate after the reaction was calculated as follows. (Cyclopentene recovery rate [%}) = (amount of recovered cyclopentene [parts])/(amount of cyclopentene in polymer or cross-linked rubber used for recovery [parts]) × 100

### <Preparation Example 1>

### (Preparation of ring-opened polymer A)

Under a nitrogen atmosphere, 100 parts of 2-norbornene (NB) as a norbornene compound, 100 parts of cyclopentene (CPE), 394 parts of toluene, and 0.12 parts of 1-hexene were placed in a glass reaction vessel equipped with a stirrer. Subsequently, 0.006 parts of dichloro-(3-phenyl-1H-inden-1-ylidene)bis(tricyclohexylphosphine) ruthenium dissolved in 10 parts of toluene was added thereto to cause a polymerization reaction at room temperature for 4 hours. After the polymerization reaction, vinyl ethyl ether was added in excess to terminate the polymerization reaction.

The polymer solution obtained above was poured into a large excess of methanol containing 2,6-di-t-butyl-p-cresol (BHT), and the precipitated polymer was collected, washed with methanol, and then vacuum dried at 50°C for 3 days to afford 160 parts of a ring-opened polymer A. The number average molecular weight (Mn) of the obtained ring-opened polymer A was 122,000, the weight average molecular weight (Mw) was 268,000, the ratio of 2-norbornene structural units/cyclopentene structural units was 62/38 (weight ratio), and the glass transition temperature (Tg) was -25°C.

### <Preparation Example 2>

### (Preparation of ring-opened polymer B)

Under a nitrogen atmosphere, 72 parts of dicyclopentadiene (DCPD) as a norbornene compound, 188 parts of cyclopentene (CPE), 1160 parts of toluene, and 0.17 parts of 1-hexene were placed in a glass reaction vessel equipped with a stirrer. Subsequently, 0.028 parts of dichloro-(3-phenyl-1H-inden-1-ylidene)bis(tricyclohexylphosphine) ruthenium dissolved in 10 parts of toluene was added thereto to cause a polymerization reaction at room temperature for 4 hours. After the polymerization reaction, vinyl ethyl ether was added in excess to terminate the polymerization reaction.

The polymer solution obtained above was poured into a large excess of methanol containing 2,6-di-t-butyl-p-cresol (BHT), and the precipitated polymer was collected, washed with methanol, and then vacuum dried at 50°C for 3 days to afford 217 parts of a ring-opened polymer B. The number average molecular weight (Mn) of the obtained ring-opened polymer B was 120,000, the weight average molecular weight (Mw) was 248,000, the ratio of dicyclopentadiene structural units/cyclopentene structural units was 36/64 (weight ratio), and the glass transition temperature (Tg) was -45°C.

### <Preparation Example 3>

### (Preparation of ring-opened polymer C)

Under a nitrogen atmosphere, 200 parts of cyclopentene (CPE), 394 parts of toluene, and 0.17 parts of parts of 1-hexene were placed in a glass reaction vessel equipped with a stirrer. Subsequently, 0.006 parts of dichloro-(3-phenyl-1H-inden-1-ylidene)bis(tricyclohexylphosphine)ruthenium dissolved in 10 parts of toluene was added thereto to cause a polymerization reaction at room temperature for 4 hours. After the polymerization reaction, vinyl ethyl ether was added in excess to terminate the polymerization reaction.

The polymer solution obtained above was poured into a large excess of methanol containing 2,6-di-t-butyl-p-cresol (BHT), and the precipitated polymer was collected, washed with methanol, and then vacuum dried at 50°C for 3 days to afford 110 parts of a ring-opened polymer C. The number average molecular weight (Mn) of the obtained ring-opened polymer C was 115,000, the weight average molecular weight (Mw) was 235,000, and the glass transition temperature (Tg) was -100°C.

### <Preparation Example 4>

### (Preparation of ring-opened polymer D)

Under a nitrogen atmosphere, 41 parts of 2-norbornene (NB) as a norbornene compound, 39 parts of dicyclopentadiene (DCPD) as a norbornene compound, 100 parts of cyclopentene (CPE), 394 parts of toluene, and 0.14 parts of 1-hexene were placed in a glass reaction vessel equipped with a stirrer. Subsequently, 0.017 parts of dichloro-(3-phenyl-1H-inden-1-ylidene)bis(tricyclohexylphosphine) ruthenium dissolved in 10 parts of toluene was added thereto to cause a polymerization reaction at room temperature for 4 hours. After the polymerization reaction, vinyl ethyl ether was added in excess to terminate the polymerization reaction.

The polymer solution obtained above was poured into a large excess of methanol containing 2,6-di-t-butyl-p-cresol (BHT), and the precipitated polymer was collected, washed with methanol, and then vacuum dried at 50°C for 3 days to afford 145 parts of a ring-opened polymer D. The number average molecular weight (Mn) of the obtained ring-opened polymer D was 126,000, the weight average molecular weight (Mw) was 254,000, the ratio of the total amount of 2-norbornene structural units and dicyclopentadiene structural units/cyclopentene structural units was 52/48 (weight ratio), and the glass transition temperature (Tg) was -40°C.

### <Preparation Example 5>

### (Preparation of ring-opened polymer E)

Under a nitrogen atmosphere, 65 parts of 1,4-methano-1,4,4a,9a-tetrahydro-9H-fluorene (MTF) as a norbornene compound, 84 parts of cyclopentene (CPE), 400 parts of toluene, and 0.15 parts of 1-hexene were placed in a glass reaction vessel equipped with a stirrer. Subsequently, 0.5 parts of (2,3,5,6-tetraphenylphenoxy)2,6-dimethylphenylimidetungsten(2,5-dimethylpyrrolide) (neophylidene) dissolved in 10 parts of toluene was added thereto to cause a polymerization reaction at 50°C for 4 hours. After the polymerization reaction, methanol was added in excess to terminate the polymerization reaction.

The polymer solution obtained above was poured into a large excess of methanol containing 2,6-di-t-butyl-p-cresol (BHT), and the precipitated polymer was collected, washed with methanol, and then vacuum dried at 50°C for 3 days to afford 135 parts of a ring-opened polymer E. The number average molecular weight (Mn) of the obtained ring-opened polymer E was 121,000, the weight average molecular weight (Mw) was 244,000, the ratio of 1,4-methano-1,4,4a,9a-tetrahydro-9H-fluorene structural units/cyclopentene structural units was 51/49 (weight ratio), and the glass transition temperature (Tg) was -23°C.

### <Preparation Example 6>

### (Preparation of ring-opened polymer F)

Under a nitrogen atmosphere, 100 parts of dicyclopentadiene (DCPD) as a norbornene compound and 400 parts of toluene were placed in a glass reaction vessel equipped with a stirrer. Subsequently, 0.9 parts of (2,3,5,6-tetraphenylphenoxy)2,6-dimethylphenylimidetungsten(2,5-dimethylpyrrolide)(neophylidene) dissolved in 10 parts of toluene was added thereto to cause a polymerization reaction at 50°C for 2 hours. Thereafter, 100 parts of cyclopentene (CPE) was added thereto to cause a polymerization reaction at 50°C for another 3 hours. After the polymerization reaction, methanol was added in excess to terminate the polymerization reaction.

The polymer solution obtained above was poured into a large excess of methanol containing 2,6-di-t-butyl-p-cresol (BHT), and the precipitated polymer was collected, washed with methanol, and then vacuum dried at 50°C for 3 days to afford 155 parts of a ring-opened polymer F. The number average molecular weight (Mn) of the obtained ring-opened polymer F was 157,000, the weight average molecular weight (Mw) was 184,000, the ratio of dicyclopentadiene structural units/cyclopentene structural units was 65/35 (weight ratio), and the glass transition temperature (Tg) was -100°C and 150°C.

### <Preparation Example 7>

### (Preparation of cross-linked rubber A)

100 Parts of the ring-opened polymer A obtained in Preparation Example 1 was masticated using a 250-ml Banbury mixer. Next, 50 parts of carbon black (product name "SEAST 9H", available from TOKAI CARBON CO., LTD.), 3 parts of zinc oxide (zinc flower No. 1), 2.0 parts of stearic acid (product name "SA-300", available from ADEKA CORPORATION), and 2.0 parts of N-phenyl-N'-(1,3-dimethylbutyl)-p-phenylenediamine (product name "NOCLAK 6C", an antioxidant, available from Ouchi Shinko Chemical Industrial Co., LTD.) were added thereto, and the mixture was kneaded at a starting temperature of 80°C for 4 minutes. The resulting rubber composition was discharged from the Banbury mixer. Next, in an open roll mixer at 50°C, the rubber composition obtained, 1.75 parts of sulfur, and 1 part of N-cyclohexylbenzothiazole-2-sulfen amide (product name "NOCCELER CZ-G (CZ)", a cross-linking accelerator, available from Ouchi Shinko Chemical Industrial Co., LTD.) were kneaded to prepare a cross-linkable rubber composition, and then crosslinking was carried out by heating at 160°C for 12 minutes to afford a cross-linked rubber A in a sheet form with a thickness of 1 mm.

### <Preparation Example 8>

### (Preparation of cross-linked rubber B)

A rubber composition and a cross-linkable rubber composition were prepared in the same manner as in Preparation Example 7 except that the ring-opened polymer B obtained in Preparation Example 2 was used instead of the ring-opened polymer A obtained in Preparation Example 1, and crosslinking was carried out in the same manner as in Preparation Example 7 to afford a cross-linked rubber B in a sheet form with a thickness of 1 mm.

### <Preparation Example 9>

### (Preparation of cross-linked rubber C)

A rubber composition and a cross-linkable rubber composition were prepared in the same manner as in Preparation Example 7 except that the ring-opened polymer C obtained in Preparation Example 3 was used instead of the ring-opened polymer A obtained in Preparation Example 1, and crosslinking was carried out in the same manner as in Preparation Example 7 to afford a cross-linked rubber C in a sheet form with a thickness of 1 mm.

### <Preparation Example 10>

### (Preparation of cross-linked rubber D)

A rubber composition and a cross-linkable rubber composition were prepared in the same manner as in Preparation Example 7 except that the ring-opened polymer D obtained in Preparation Example 4 was used instead of the ring-opened polymer A obtained in Preparation Example 1, and crosslinking was carried out in the same manner as in Preparation Example 7 to afford a cross-linked rubber D in a sheet form with a thickness of 1 mm.

### <Preparation Example 11>

### (Preparation of cross-linked rubber E)

A rubber composition and a cross-linkable rubber composition were prepared in the same manner as in Preparation Example 7 except that the ring-opened polymer E obtained in Preparation Example 5 was used instead of the ring-opened polymer A obtained in Preparation Example 1, and crosslinking was carried out in the same manner as in Preparation Example 7 to afford a cross-linked rubber E in a sheet form with a thickness of 1 mm.

### <Preparation Example 12>

### (Preparation of cross-linked rubber F)

A rubber composition and a cross-linkable rubber composition were prepared in the same manner as in Preparation Example 7 except that the ring-opened polymer F obtained in Preparation Example 6 was used instead of the ring-opened polymer A obtained in Preparation Example 1, and crosslinking was carried out in the same manner as in Preparation Example 7 to afford a cross-linked rubber F in a sheet form with a thickness of 1 mm.

### <Example 1>

Under a nitrogen atmosphere, 50 parts of the ring-opened polymer A obtained in Preparation Example 1 and 500 parts of toluene (0.577 L) were placed in a glass reaction vessel equipped with a stirrer, stirred, and dissolved. Then, a solution of 0.08 parts of benzylidene(1,3-dimesitylimidazolidin-2-ylidene) (tricyclohexylphosphine)ruthenium dichloride dissolved in 10 parts of toluene was added thereto, and a depolymerization reaction was carried out with stirring at room temperature (25°C) for 2 hours. After the reaction, vinyl ethyl ether was added in excess to terminate the depolymerization.

When the amount of cyclopentene contained in the solution after the reaction was quantified by gas chromatography, 15.2 parts of cyclopentene was produced (the concentration of cyclopentene in the cyclopentene solution (cyclopentene solution in toluene) was 0.387 mol/L (concentration at 25°C, the same applies hereinafter)), and the cyclopentene recovery rate was 80%. The number average molecular weight (Mn) of the ring-opened polymer determined by GPC measurement of the soluble part was 71,000, and the weight average molecular weight (Mw) was 128,000. Further, the ratio of 2-norbornene structural units/cyclopentene structural units determined by ¹H-NMR was 89/11 (weight ratio), which was in good agreement with the result obtained by gas chromatography (i.e., the amount of recovered cyclopentene).

### <Example 2>

Under a nitrogen atmosphere, 50 parts of the ring-opened polymer B obtained in Preparation Example 2 and 500 parts of toluene were placed in a glass reaction vessel equipped with a stirrer, stirred, and dissolved. Then, a solution of 0.1 parts of benzylidene(1,3-dimesitylimidazolidin-2-ylidene) (tricyclohexylphosphine)ruthenium dichloride dissolved in 10 parts of toluene was added thereto, and a depolymerization reaction was carried out with stirring at room temperature (25°C) for 2 hours. After the reaction, vinyl ethyl ether was added in excess to terminate the depolymerization.

When the amount of cyclopentene contained in the solution after the reaction was quantified by gas chromatography, 25.6 parts of cyclopentene was produced (the concentration of cyclopentene in the cyclopentene solution was 0.651 mol/L), and the cyclopentene recovery rate was 80%. The number average molecular weight (Mn) of the ring-opened polymer determined by GPC measurement of the soluble part was 18,000, and the weight average molecular weight (Mw) was 41,000. Further, the ratio of dicyclopentadiene structural units/cyclopentene structural units determined by ¹H-NMR was 74/26 (weight ratio), which was in good agreement with the result obtained by gas chromatography (i.e., the amount of recovered cyclopentene).

### <Example 3>

Under a nitrogen atmosphere, 50 parts of the ring-opened polymer B obtained in Preparation Example 2 and 100 parts of toluene were placed in a glass reaction vessel equipped with a stirrer, stirred, and dissolved. Then, a solution of 0.1 parts of benzylidene(1,3-dimesitylimidazolidin-2-ylidene) (tricyclohexylphosphine)ruthenium dichloride dissolved in 10 parts of toluene was added thereto, and a depolymerization reaction was carried out with stirring at room temperature (25°C) for 2 hours. After the reaction, vinyl ethyl ether was added in excess to terminate the depolymerization.

When the amount of cyclopentene contained in the solution after the reaction was quantified by gas chromatography, 9.3 parts of cyclopentene was produced (the concentration of cyclopentene in the cyclopentene solution was 1.183 mol/L), and the cyclopentene recovery rate was 29%. The number average molecular weight (Mn) of the ring-opened polymer determined by GPC measurement of the soluble part was 99,000, and the weight average molecular weight (Mw) was 205,000. Further, the ratio of dicyclopentadiene structural units/cyclopentene structural units determined by ¹H-NMR was 43/57 (weight ratio), which was in good agreement with the result obtained by gas chromatography (i.e., the amount of recovered cyclopentene).

### <Example 4>

Under a nitrogen atmosphere, 50 parts of the ring-opened polymer D obtained in Preparation Example 4 and 500 parts of toluene were placed in a glass reaction vessel equipped with a stirrer, stirred, and dissolved. Then, a solution of 0.1 parts of benzylidene(1,3-dimesitylimidazolidin-2-ylidene) (tricyclohexylphosphine)ruthenium dichloride dissolved in 10 parts of toluene was added thereto, and a depolymerization reaction was carried out with stirring at room temperature (25°C) for 2 hours. After the reaction, vinyl ethyl ether was added in excess to terminate the depolymerization.

When the amount of cyclopentene contained in the solution after the reaction was quantified by gas chromatography, 17.9 parts of cyclopentene was produced (the concentration of cyclopentene in the cyclopentene solution was 0.455 mol/L), and the cyclopentene recovery rate was 75%. The number average molecular weight (Mn) of the ring-opened polymer determined by GPC measurement of the soluble part was 45,000, and the weight average molecular weight (Mw) was 87,000. Further, the ratio of the total amount of 2-norbornene structural units and dicyclopentadiene structural units/cyclopentene structural units determined by ¹H-NMR was 81/19 (weight ratio), which was in good agreement with the result obtained by gas chromatography (i.e., the amount of recovered cyclopentene).

### <Example 5>

Under a nitrogen atmosphere, 50 parts of the ring-opened polymer E obtained in Preparation Example 5 and 500 parts of toluene were placed in a glass reaction vessel equipped with a stirrer, stirred, and dissolved. Then, a solution of 0.1 parts of benzylidene(1,3-dimesitylimidazolidin-2-ylidene) (tricyclohexylphosphine)ruthenium dichloride dissolved in 10 parts of toluene was added thereto, and a depolymerization reaction was carried out with stirring at room temperature (25°C) for 2 hours. After the reaction, vinyl ethyl ether was added in excess to terminate the depolymerization.

When the amount of cyclopentene contained in the solution after the reaction was quantified by gas chromatography, 18.4 parts of cyclopentene was produced (the concentration of cyclopentene in the cyclopentene solution was 0.468 mol/L), and the cyclopentene recovery rate was 75%. The number average molecular weight (Mn) of the ring-opened polymer determined by GPC measurement of the soluble part was 42,000, and the weight average molecular weight (Mw) was 86,000. Further, the ratio of 1,4-methano-1,4,4a,9a-tetrahydro-9H-fluorene structural units/cyclopentene structural units determined by ¹H-NMR was 84/16 (weight ratio), which was in good agreement with the result obtained by gas chromatography (i.e., the amount of recovered cyclopentene).

### <Example 6>

Under a nitrogen atmosphere, 80 parts of the cross-linked rubber A (50 parts as the amount of organic polymers), which was obtained in Preparation Example 7 and cut into small pieces with a dimension of 1 mm × 2 mm × 2 mm, and 500 parts of toluene were placed in a glass reaction vessel equipped with a stirrer, and stirred. Then, a solution of 1.2 parts of benzylidene(1,3-dimesitylimidazolidin-2-ylidene)(tricyclohexylphosphine)ruthenium dichloride dissolved in 10 parts of toluene was added thereto, and a depolymerization reaction was carried out with stirring at room temperature (25°C) for 2 hours. The cross-linked rubber A in the form of small pieces with a dimension of 1 mm × 2 mm × 2 mm lost their shape as the depolymerization reaction proceeded, and was dissolved and dispersed in toluene to form black precipitates of carbon. After the reaction, vinyl ethyl ether was added in excess to terminate the depolymerization.

When the amount of cyclopentene contained in the mixture after the reaction was quantified by gas chromatography, 13.1 parts of cyclopentene was produced (the concentration of cyclopentene in the cyclopentene solution was 0.333 mol/L), and the cyclopentene recovery rate was 69%. The number average molecular weight (Mn) of the ring-opened polymer determined by GPC measurement of the soluble part was 90,000, and the weight average molecular weight (Mw) was 183,000. Further, the ratio of 2-norbornene structural units/cyclopentene structural units determined by ¹H-NMR was 84/16 (weight ratio), which was in good agreement with the result obtained by gas chromatography (i.e., the amount of recovered cyclopentene).

### <Example 7>

Under a nitrogen atmosphere, 80 parts of the cross-linked rubber B (50 parts as the amount of organic polymers), which was obtained in Preparation Example 8 and cut into small pieces with a dimension of 1 mm × 2 mm × 2 mm, and 500 parts of toluene were placed in a glass reaction vessel equipped with a stirrer, and stirred. Then, a solution of 1.4 parts of benzylidene(1,3-dimesitylimidazolidin-2-ylidene)(tricyclohexylphosphine)ruthenium dichloride dissolved in 10 parts of toluene was added thereto, and a depolymerization reaction was carried out with stirring at room temperature (25°C) for 2 hours. The cross-linked rubber B in the form of small pieces with a dimension of 1 mm × 2 mm × 2 rrun lost their shape as the depolymerization reaction proceeded, and was dissolved and dispersed in toluene to form black precipitates of carbon. After the reaction, vinyl ethyl ether was added in excess to terminate the depolymerization.

When the amount of cyclopentene contained in the mixture after the reaction was quantified by gas chromatography, 22.5 parts of cyclopentene was produced (the concentration of cyclopentene in the cyclopentene solution was 0.573 mol/L), and the cyclopentene recovery rate was 70%. The number average molecular weight (Mn) of the ring-opened polymer determined by GPC measurement of the soluble part was 66,000, and the weight average molecular weight (Mw) was 136,000. Further, the ratio of dicyclopentadiene structural units/cyclopentene structural units determined by ¹H-NMR was 65/35 (weight ratio), which was in good agreement with the result obtained by gas chromatography (i.e., the amount of recovered cyclopentene).

### <Example 8>

Under a nitrogen atmosphere, 80 parts of the cross-linked rubber B (50 parts as the amount of organic polymers), which was obtained in Preparation Example 8 and cut into small pieces with a dimension of 1 mm × 2 mm × 2 mm, and 100 parts of toluene were placed in a glass reaction vessel equipped with a stirrer, and stirred. Then, a solution of 1.4 parts of benzylidene(1,3-dimesitylimidazolidin-2-ylidene) (tricyclohexylphosphine) ruthenium dichloride dissolved in 10 parts of toluene was added thereto, and a depolymerization reaction was carried out with stirring at room temperature (25°C) for 2 hours. The cross-linked rubber B in the form of small pieces with a dimension of 1 mm × 2 mm × 2 mm lost their shape as the depolymerization reaction proceeded, and was dissolved and dispersed in toluene to form black precipitates of carbon. After the reaction, vinyl ethyl ether was added in excess to terminate the depolymerization.

When the amount of cyclopentene contained in the mixture after the reaction was quantified by gas chromatography, 8.6 parts of cyclopentene was produced (the concentration of cyclopentene in the cyclopentene solution was 1.094 mol/L), and the cyclopentene recovery rate was 27%. The number average molecular weight (Mn) of the ring-opened polymer determined by GPC measurement of the soluble part was 99,000, and the weight average molecular weight (Mw) was 212,000. Further, the ratio of dicyclopentadiene structural units/cyclopentene structural units determined by ¹H-NMR was 42/58 (weight ratio), which was in good agreement with the result obtained by gas chromatography (i.e., the amount of recovered cyclopentene).

### <Example 9>

Under a nitrogen atmosphere, 80 parts of the cross-linked rubber C (50 parts as the amount of organic polymers), which was obtained in Preparation Example 9 and cut into small pieces with a dimension of 1 mm × 2 mm × 2 mm, and 500 parts of toluene were placed in a glass reaction vessel equipped with a stirrer, and stirred. Then, a solution of 0.5 parts of benzylidene(1,3-dimesitylimidazolidin-2-ylidene)(tricyclohexylphosphine)ruthenium dichloride dissolved in 10 parts of toluene was added thereto, and a depolymerization reaction was carried out with stirring at room temperature (25°C) for 2 hours. The cross-linked rubber C in the form of small pieces with a dimension of 1 mm × 2 mm × 2 mm lost their shape as the depolymerization reaction proceeded, and was dissolved and dispersed in toluene to form black precipitates of carbon. After the reaction, vinyl ethyl ether was added in excess to terminate the depolymerization.

When the amount of cyclopentene contained in the mixture after the reaction was quantified by gas chromatography, 44.3 parts of cyclopentene was produced (the concentration of cyclopentene in the cyclopentene solution was 1.127 mol/L), and the cyclopentene recovery rate was 89%. No peak corresponding to a high molecular weight was observed by GPC measurement of the soluble part.

### <Example 10>

Under a nitrogen atmosphere, 80 parts of the cross-linked rubber C (50 parts as the amount of organic polymers), which was obtained in Preparation Example 9 and cut into small pieces with a dimension of 1 mm × 2 rrun × 2 mm, and 100 parts of toluene were placed in a glass reaction vessel equipped with a stirrer, and stirred. Then, a solution of 0.5 parts of benzylidene(1,3-dimesitylimidazolidin-2-ylidene) (tricyclohexylphosphine) ruthenium dichloride dissolved in 10 parts of toluene was added thereto, and a depolymerization reaction was carried out with stirring at room temperature (25°C) for 2 hours. The cross-linked rubber C in the form of small pieces with a dimension of 1 mm × 2 mm × 2 rrun lost their shape as the depolymerization reaction proceeded, and was dissolved and dispersed in toluene to form black precipitates of carbon. After the reaction, vinyl ethyl ether was added in excess to terminate the depolymerization.

When the amount of cyclopentene contained in the mixture after the reaction was quantified by gas chromatography, 11.5 parts of cyclopentene was produced (the concentration of cyclopentene in the cyclopentene solution was 1.463 mol/L), and the cyclopentene recovery rate was 23%. The number average molecular weight (Mn) of the ring-opened polymer determined by GPC measurement of the soluble part was 86,000, and the weight average molecular weight (Mw) was 176,000. Further, the ratio of cyclopentene structural units determined by ¹H-NMR was 100 (weight ratio).

### <Example 11>

Under a nitrogen atmosphere, 80 parts of the cross-linked rubber D (50 parts as the amount of organic polymers), which was obtained in Preparation Example 10 and cut into small pieces with a dimension of 1 mm × 2 mm × 2 mm, and 500 parts of toluene were placed in a glass reaction vessel equipped with a stirrer, and stirred. Then, a solution of 1.4 parts of benzylidene(1,3-dimesitylimidazolidin-2-ylidene) (tricyclohexylphosphine) ruthenium dichloride dissolved in 10 parts of toluene was added thereto, and a depolymerization reaction was carried out with stirring at room temperature (25°C) for 2 hours. The cross-linked rubber D in the form of small pieces with a dimension of 1 mm × 2 mm × 2 rrun lost their shape as the depolymerization reaction proceeded, and was dissolved and dispersed in toluene to form black precipitates of carbon. After the reaction, vinyl ethyl ether was added in excess to terminate the depolymerization.

When the amount of cyclopentene contained in the mixture after the reaction was quantified by gas chromatography, 15.8 parts of cyclopentene was produced (the concentration of cyclopentene in the cyclopentene solution was 0.402 mol/L), and the cyclopentene recovery rate was 66%. The number average molecular weight (Mn) of the ring-opened polymer determined by GPC measurement of the soluble part was 75,000, and the weight average molecular weight (Mw) was 158,000. Further, the ratio of the total amount of 2-norbornene structural units and dicyclopentadiene structural units/cyclopentene structural units determined by ¹H-NMR was 76/24 (weight ratio), which was in good agreement with the result obtained by gas chromatography (i.e., the amount of recovered cyclopentene).

### <Example 12>

Under a nitrogen atmosphere, 80 parts of the cross-linked rubber E (50 parts as the amount of organic polymers), which was obtained in Preparation Example 11 and cut into small pieces with a dimension of 1 mm × 2 mm × 2 mm, and 500 parts of toluene were placed in a glass reaction vessel equipped with a stirrer, and stirred. Then, a solution of 1.4 parts of benzylidene(1,3-dimesitylimidazolidin-2-ylidene)(tricyclohexylphosphine)ruthenium dichloride dissolved in 10 parts of toluene was added thereto, and a depolymerization reaction was carried out with stirring at room temperature (25°C) for 2 hours. The cross-linked rubber E in the form of small pieces with a dimension of 1 mm × 2 mm × 2 mm lost their shape as the depolymerization reaction proceeded, and was dissolved and dispersed in toluene to form black precipitates of carbon. After the reaction, vinyl ethyl ether was added in excess to terminate the depolymerization.

When the amount of cyclopentene contained in the mixture after the reaction was quantified by gas chromatography, 14.6 parts of cyclopentene was produced (the concentration of cyclopentene in the cyclopentene solution was 0.372 mol/L), and the cyclopentene recovery rate was 60%. The number average molecular weight (Mn) of the ring-opened polymer determined by GPC measurement of the soluble part was 82,000, and the weight average molecular weight (Mw) was 141,000. Further, the ratio of 1,4-methano-1,4,4a,9a-tetrahydro-9H-fluorene structural units/cyclopentene structural units determined by ¹H-NMR was 72/28 (weight ratio), which was in good agreement with the result obtained by gas chromatography (i.e., the amount of recovered cyclopentene).

### <Example 13>

Under a nitrogen atmosphere, 80 parts of the cross-linked rubber A (50 parts as the amount of organic polymers), which was obtained in Preparation Example 7 and cut into small pieces with a dimension of 1 mm × 2 rrun × 2 mm, and 500 parts of toluene were placed in a glass reaction vessel equipped with a stirrer, and stirred. Then, a solution of 2.2 parts of (2,3,5,6-tetraphenylphenoxy)2,6-dimethylphenylimidetungsten(2,5-dimethylpyrrolide) (neophylidene) dissolved in 10 parts of toluene was added thereto, and a depolymerization reaction was carried out with stirring at room temperature (25°C) for 2 hours. The cross-linked rubber A in the form of small pieces with a dimension of 1 mm × 2 mm × 2 mm lost their shape as the depolymerization reaction proceeded, and was dissolved and dispersed in toluene to form black precipitates of carbon. After the reaction, vinyl ethyl ether was added in excess to terminate the depolymerization.

When the amount of cyclopentene contained in the mixture after the reaction was quantified by gas chromatography, 11.6 parts of cyclopentene was produced (the concentration of cyclopentene in the cyclopentene solution was 0.295 mol/L), and the cyclopentene recovery rate was 61%. The number average molecular weight (Mn) of the ring-opened polymer determined by GPC measurement of the soluble part was 96,000, and the weight average molecular weight (Mw) was 184,000. Further, the ratio of 2-norbornene structural units/cyclopentene structural units determined by ¹H-NMR was 80/20 (weight ratio), which was in good agreement with the result obtained by gas chromatography (i.e., the amount of recovered cyclopentene).

### <Example 14>

Under a nitrogen atmosphere, 80 parts of the cross-linked rubber A (50 parts as the amount of organic polymers), which was obtained in Preparation Example 7 and cut into small pieces with a dimension of 1 mm × 2 mm × 2 mm, and 500 parts of toluene were placed in a glass reaction vessel equipped with a stirrer, and stirred. Then, a solution of 1.4 parts of phenylimidotungsten tetrachloride tetrahydrofuran and 1.1 parts of diethylaluminum ethoxide dissolved in 20 parts of toluene was added thereto, and a depolymerization reaction was carried out with stirring at room temperature (25°C) for 2 hours. The cross-linked rubber A in the form of small pieces with a dimension of 1 mm × 2 mm × 2 mm lost their shape as the depolymerization reaction proceeded, and was dissolved and dispersed in toluene to form black precipitates of carbon. After the reaction, methanol was added in excess to terminate the depolymerization.

When the amount of cyclopentene contained in the mixture after the reaction was quantified by gas chromatography, 11.4 parts of cyclopentene was produced (the concentration of cyclopentene in the cyclopentene solution was 0.290 mol/L), and the cyclopentene recovery rate was 60%. The number average molecular weight (Mn) of the ring-opened polymer determined by GPC measurement of the soluble part was 98,000, and the weight average molecular weight (Mw) was 189,000. Further, the ratio of 2-norbornene structural units/cyclopentene structural units determined by ¹H-NMR was 79/21 (weight ratio), which was in good agreement with the result obtained by gas chromatography (i.e., the amount of recovered cyclopentene).

### <Example 15>

Under a nitrogen atmosphere, 50 parts of the ring-opened polymer F obtained in Preparation Example 6 and 500 parts of toluene were placed in a glass reaction vessel equipped with a stirrer, stirred, and dissolved. Then, a solution of 0.1 parts of benzylidene(1,3-dimesitylimidazolidin-2-ylidene) (tricyclohexylphosphine)ruthenium dichloride dissolved in 10 parts of toluene was added thereto, and a depolymerization reaction was carried out with stirring at room temperature (25°C) for 2 hours. After the reaction, vinyl ethyl ether was added in excess to terminate the depolymerization.

When the amount of cyclopentene contained in the solution after the reaction was quantified by gas chromatography, 17.2 parts of cyclopentene was produced (the concentration of cyclopentene in the cyclopentene solution was 0.438 mol/L), and the cyclopentene recovery rate was 97%. The number average molecular weight (Mn) of the ring-opened polymer determined by GPC measurement of the soluble part was 105,000, and the weight average molecular weight (Mw) was 123,000. Further, the ratio of dicyclopentadiene structural units/cyclopentene structural units determined by ¹H-NMR was 100/0 (weight ratio), which was in good agreement with the result obtained by gas chromatography (i.e., the amount of recovered cyclopentene).

### <Example 16>

Under a nitrogen atmosphere, 80 parts of the cross-linked rubber F (50 parts as the amount of organic polymers), which was obtained in Preparation Example 12 and cut into small pieces with a dimension of 1 mm × 2 mm × 2 mm, and 500 parts of toluene were placed in a glass reaction vessel equipped with a stirrer, and stirred. Then, a solution of 0.5 parts of benzylidene(1,3-dimesitylimidazolidin-2-ylidene) (tricyclohexylphosphine) ruthenium dichloride dissolved in 10 parts of toluene was added thereto, and a depolymerization reaction was carried out with stirring at room temperature (25°C) for 2 hours. The cross-linked rubber F in the form of small pieces with a dimension of 1 mm × 2 mm × 2 mm lost their shape as the depolymerization reaction proceeded, and was dissolved and dispersed in toluene to form black precipitates of carbon. After the reaction, ethyl vinyl ether was added in excess to terminate the depolymerization.

When the amount of cyclopentene contained in the mixture after the reaction was quantified by gas chromatography, 14.1 parts of cyclopentene was produced (the concentration of cyclopentene in the cyclopentene solution was 0.359 mol/L), and the cyclopentene recovery rate was 79%. The number average molecular weight (Mn) of the ring-opened polymer determined by GPC measurement of the soluble part was 117,000, and the weight average molecular weight (Mw) was 149,000. Further, the ratio of dicyclopentadiene structural units/cyclopentene structural units determined by ¹H-NMR was 91/9 (weight ratio), which was in good agreement with the result obtained by gas chromatography (i.e., the amount of recovered cyclopentene).

### <Example 17>

Under a nitrogen atmosphere, 50 parts of the ring-opened polymer A obtained in Preparation Example 1 and 500 parts of toluene (0.577 L) were placed in a glass reaction vessel equipped with a stirrer, stirred, and dissolved. Then, a dispersion of 6.23 parts of benzylidene(1,3-dimesitylimidazolidin-2-ylidene) (tricyclohexylphosphine)ruthenium dichloride dispersed in 10 parts of toluene was added thereto, and a depolymerization reaction was carried out with stirring at 60°C for 24 hours. After the reaction, vinyl ethyl ether was added in excess to terminate the depolymerization.

When the amount of cyclopentene contained in the solution after the reaction was quantified by gas chromatography, 18.2 parts of cyclopentene was produced (the concentration of cyclopentene in the cyclopentene solution (cyclopentene solution in toluene) was 0.464 mol/L), and the cyclopentene recovery rate was 96%. The number average molecular weight (Mn) of the ring-opened polymer determined by GPC measurement of the soluble part was 7,100, and the weight average molecular weight (Mw) was 15,600. Further, the ratio of 2-norbornene structural units/cyclopentene structural units determined by ¹H-NMR was 98/2 (weight ratio), which was in good agreement with the result obtained by gas chromatography (i.e., the amount of recovered cyclopentene).

### <Example 18>

Under a nitrogen atmosphere, 50 parts of the ring-opened polymer B obtained in Preparation Example 2 and 500 parts of toluene (0.577 L) were placed in a glass reaction vessel equipped with a stirrer, stirred, and dissolved. Then, a dispersion of 6.23 parts of benzylidene(1,3-dimesitylimidazolidin-2-ylidene) (tricyclohexylphosphine)ruthenium dichloride dispersed in 10 parts of toluene was added thereto, and a depolymerization reaction was carried out with stirring at 60°C for 24 hours. After the reaction, vinyl ethyl ether was added in excess to terminate the depolymerization.

When the amount of cyclopentene contained in the solution after the reaction was quantified by gas chromatography, 31.7 parts of cyclopentene was produced (the concentration of cyclopentene in the cyclopentene solution (cyclopentene solution in toluene) was 0.806 mol/L), and the cyclopentene recovery rate was 99%. The number average molecular weight (Mn) of the ring-opened polymer determined by GPC measurement of the soluble part was 810, and the weight average molecular weight (Mw) was 2,460. Further, the ratio of dicyclopentadiene structural units/cyclopentene structural units determined by ¹H-NMR was 99/1 (weight ratio), which was in good agreement with the result obtained by gas chromatography (i.e., the amount of recovered cyclopentene).

### <Example 19>

Under a nitrogen atmosphere, 50 parts of the ring-opened polymer C obtained in Preparation Example 3 and 500 parts of toluene (0.577 L) were placed in a glass reaction vessel equipped with a stirrer, stirred, and dissolved. Then, a dispersion of 6.23 parts of benzylidene(1,3-dimesitylimidazolidin-2-ylidene) (tricyclohexylphosphine)ruthenium dichloride dispersed in 10 parts of toluene was added thereto, and a depolymerization reaction was carried out with stirring at 60°C for 24 hours. After the reaction, vinyl ethyl ether was added in excess to terminate the depolymerization.

When the amount of cyclopentene contained in the solution after the reaction was quantified by gas chromatography, 50.0 parts of cyclopentene was produced (the concentration of cyclopentene in the cyclopentene solution (cyclopentene solution in toluene) was 1.272 mol/L), and the cyclopentene recovery rate was 100%. No peak corresponding to a high molecular weight was observed by GPC measurement of the soluble part.

### <Example 20>

Under a nitrogen atmosphere, 80 parts of the cross-linked rubber A (50 parts as the amount of organic polymers), which was obtained in Preparation Example 7 and cut into small pieces with a dimension of 1 mm × 2 mm × 2 mm, and 500 parts of toluene were placed in a glass reaction vessel equipped with a stirrer, and stirred. Then, a dispersion of 6.23 parts of benzylidene(1,3-dimesitylimidazolidin-2-ylidene)(tricyclohexylphosphine)ruthenium dichloride dispersed in 10 parts of toluene was added thereto, and a depolymerization reaction was carried out with stirring at 60°C for 24 hours. The cross-linked rubber A in the form of small pieces with a dimension of 1 mm × 2 mm × 2 mm lost their shape as the depolymerization reaction proceeded, and was dissolved and dispersed in toluene to form black precipitates of carbon. After the reaction, vinyl ethyl ether was added in excess to terminate the depolymerization.

When the amount of cyclopentene contained in the mixture after the reaction was quantified by gas chromatography, 15.2 parts of cyclopentene was produced (the concentration of cyclopentene in the cyclopentene solution was 0.387 mol/L), and the cyclopentene recovery rate was 80%. The number average molecular weight (Mn) of the ring-opened polymer determined by GPC measurement of the soluble part was 7,760, and the weight average molecular weight (Mw) was 15,600. Further, the ratio of 2-norbornene structural units/cyclopentene structural units determined by ¹H-NMR was 89/11 (weight ratio), which was in good agreement with the result obtained by gas chromatography (i.e., the amount of recovered cyclopentene).

### <Example 21>

Under a nitrogen atmosphere, 80 parts of the cross-linked rubber B (50 parts as the amount of organic polymers), which was obtained in Preparation Example 8 and cut into small pieces with a dimension of 1 mm × 2 mm × 2 mm, and 500 parts of toluene were placed in a glass reaction vessel equipped with a stirrer, and stirred. Then, a dispersion of 6.23 parts of benzylidene(1,3-dimesitylimidazolidin-2-ylidene) (tricyclohexylphosphine) ruthenium dichloride dispersed in 10 parts of toluene was added thereto, and a depolymerization reaction was carried out with stirring at 60°C for 24 hours. The cross-linked rubber B in the form of small pieces with a dimension of 1 mm × 2 mm × 2 mm lost their shape as the depolymerization reaction proceeded, and was dissolved and dispersed in toluene to form black precipitates of carbon. After the reaction, vinyl ethyl ether was added in excess to terminate the depolymerization.

When the amount of cyclopentene contained in the mixture after the reaction was quantified by gas chromatography, 25.6 parts of cyclopentene was produced (the concentration of cyclopentene in the cyclopentene solution was 0.651 mol/L), and the cyclopentene recovery rate was 80%. The number average molecular weight (Mn) of the ring-opened polymer determined by GPC measurement of the soluble part was 810, and the weight average molecular weight (Mw) was 3,590. Further, the ratio of dicyclopentadiene structural units/cyclopentene structural units determined by ¹H-NMR was 75/25 (weight ratio), which was in good agreement with the result obtained by gas chromatography (i.e., the amount of recovered cyclopentene).

### <Example 22>

Under a nitrogen atmosphere, 80 parts of the cross-linked rubber C (50 parts as the amount of organic polymers), which was obtained in Preparation Example 9 and cut into small pieces with a dimension of 1 mm × 2 mm × 2 mm, and 500 parts of toluene were placed in a glass reaction vessel equipped with a stirrer, and stirred. Then, a dispersion of 6.23 parts of benzylidene(1,3-dimesitylimidazolidin-2-ylidene) (tricyclohexylphosphine) ruthenium dichloride dispersed in 10 parts of toluene was added thereto, and a depolymerization reaction was carried out with stirring at 60°C for 24 hours. The cross-linked rubber C in the form of small pieces with a dimension of 1 mm × 2 mm × 2 mm lost their shape as the depolymerization reaction proceeded, and was dissolved and dispersed in toluene to form black precipitates of carbon. After the reaction, vinyl ethyl ether was added in excess to terminate the depolymerization.

When the amount of cyclopentene contained in the mixture after the reaction was quantified by gas chromatography, 49.5 parts of cyclopentene was produced (the concentration of cyclopentene in the cyclopentene solution was 1.260 mol/L), and the cyclopentene recovery rate was 99%. No peak corresponding to a high molecular weight was observed by GPC measurement of the soluble part.

### <Example 23>

Under a nitrogen atmosphere, 50 parts of the ring-opened polymer A obtained in Preparation Example 1 and 500 parts of toluene (0.577 L) were placed in a glass reaction vessel equipped with a stirrer, stirred, and dissolved. Then, a solution of 0.21 parts of bis(1,1,1,3,3,3-hexafluoro-2-propoxy)neophylidenemolybdenum(VI) (2,6-dimethylphenylimido) dissolved in 10 parts of toluene was added thereto, and a depolymerization reaction was carried out with stirring at room temperature (25°C) for 2 hours. After the reaction, methanol was added in excess to terminate the depolymerization.

When the amount of cyclopentene contained in the solution after the reaction was quantified by gas chromatography, 13.3 parts of cyclopentene was produced (the concentration of cyclopentene in the cyclopentene solution (cyclopentene solution in toluene) was 0.338 mol/L), and the cyclopentene recovery rate was 70%. The number average molecular weight (Mn) of the ring-opened polymer determined by GPC measurement of the soluble part was 83,000, and the weight average molecular weight (Mw) was 139,000. Further, the ratio of 2-norbornene structural units/cyclopentene structural units determined by ¹H-NMR was 85/15 (weight ratio), which was in good agreement with the result obtained by gas chromatography (i.e., the amount of recovered cyclopentene).

### <Example 24>

Under a nitrogen atmosphere, 50 parts of the ring-opened polymer B obtained in Preparation Example 2 and 500 parts of toluene (0.577 L) were placed in a glass reaction vessel equipped with a stirrer, stirred, and dissolved. Then, a solution of 0.21 parts of bis(1,1,1,3,3,3-hexafluoro-2-propoxy)neophylidenemolybdenum(VI) (2,6-dimethylphenylimido) dissolved in 10 parts of toluene was added thereto, and a depolymerization reaction was carried out with stirring at room temperature (25°C) for 2 hours. After the reaction, methanol was added in excess to terminate the depolymerization.

When the amount of cyclopentene contained in the solution after the reaction was quantified by gas chromatography, 24.0 parts of cyclopentene was produced (the concentration of cyclopentene in the cyclopentene solution (cyclopentene solution in toluene) was 0.611 mol/L), and the cyclopentene recovery rate was 75%. The number average molecular weight (Mn) of the ring-opened polymer determined by GPC measurement of the soluble part was 55,000, and the weight average molecular weight (Mw) was 97,000. Further, the ratio of dicyclopentadiene structural units/cyclopentene structural units determined by ¹H-NMR was 70/30 (weight ratio), which was in good agreement with the result obtained by gas chromatography (i.e., the amount of recovered cyclopentene).

### <Example 25>

Under a nitrogen atmosphere, 50 parts of the ring-opened polymer C obtained in Preparation Example 3 and 500 parts of toluene (0.577 L) were placed in a glass reaction vessel equipped with a stirrer, stirred, and dissolved. Then, a solution of 0.21 parts of bis(1,1,1,3,3,3-hexafluoro-2-propoxy)neophylidenemolybdenum(VI) (2,6-dimethylphenylimido) dissolved in 10 parts of toluene was added thereto, and a depolymerization reaction was carried out with stirring at room temperature (25°C) for 2 hours. After the reaction, methanol was added in excess to terminate the depolymerization.

When the amount of cyclopentene contained in the solution after the reaction was quantified by gas chromatography, 50.0 parts of cyclopentene was produced (the concentration of cyclopentene in the cyclopentene solution (cyclopentene solution in toluene) was 1.272 mol/L), and the cyclopentene recovery rate was 100%. No peak corresponding to a high molecular weight was observed by GPC measurement of the soluble part.

### <Example 26>

Under a nitrogen atmosphere, 5.43 parts of bis(1,1,1,3,3,3-hexafluoro-2-propoxy)neophylidenemolybdenum(VI) (2,6-dimethylphenylimido) and 510 parts of toluene were placed in a glass reaction vessel equipped with a stirrer, and stirred. Then, 80 parts of the cross-linked rubber A (50 parts as the amount of organic polymers), which was obtained in Preparation Example 7 and cut into small pieces with a dimension of 1 mm × 2 mm × 2 mm, was added thereto, and a depolymerization reaction was carried out with stirring at 50°C for 24 hours. The cross-linked rubber A in the form of small pieces with a dimension of 1 mm × 2 mm × 2 mm lost their shape as the depolymerization reaction proceeded, and was dissolved and dispersed in toluene to form black precipitates of carbon. After the reaction, methanol was added in excess to terminate the depolymerization.

When the amount of cyclopentene contained in the solution after the reaction was quantified by gas chromatography, 14.4 parts of cyclopentene was produced (the concentration of cyclopentene in the cyclopentene solution was 0.367 mol/L), and the cyclopentene recovery rate was 76%. The number average molecular weight (Mn) of the ring-opened polymer determined by GPC measurement of the soluble part was 16,700, and the weight average molecular weight (Mw) was 33,900. Further, the ratio of 2-norbornene structural units/cyclopentene structural units determined by ¹H-NMR was 86/14 (weight ratio), which was in good agreement with the result obtained by gas chromatography (i.e., the amount of recovered cyclopentene).

### <Example 27>

Under a nitrogen atmosphere, 5.43 parts of bis(1,1,1,3,3,3-hexafluoro-2-propoxy)neophylidenemolybdenum(VI) (2,6-dimethylphenylimido) and 510 parts of toluene were placed in a glass reaction vessel equipped with a stirrer, and stirred. Then, 80 parts of the cross-linked rubber B (50 parts as the amount of organic polymers), which was obtained in Preparation Example 8 and cut into small pieces with a dimension of 1 mm × 2 mm × 2 mm, was added thereto, and a depolymerization reaction was carried out with stirring at 50°C for 24 hours. The cross-linked rubber B in the form of small pieces with a dimension of 1 mm × 2 mm × 2 mm lost their shape as the depolymerization reaction proceeded, and was dissolved and dispersed in toluene to form black precipitates of carbon. After the reaction, methanol was added in excess to terminate the depolymerization.

When the amount of cyclopentene contained in the solution after the reaction was quantified by gas chromatography, 25.6 parts of cyclopentene was produced (the concentration of cyclopentene in the cyclopentene solution was 0.651 mol/L), and the cyclopentene recovery rate was 80%. The number average molecular weight (Mn) of the ring-opened polymer determined by GPC measurement of the soluble part was 820, and the weight average molecular weight (Mw) was 4,200. Further, the ratio of dicyclopentadiene structural units/cyclopentene structural units determined by ¹H-NMR was 75/25 (weight ratio), which was in good agreement with the result obtained by gas chromatography (i.e., the amount of recovered cyclopentene).

### <Example 28>

Under a nitrogen atmosphere, 5.43 parts of bis(1,1,1,3,3,3-hexafluoro-2-propoxy)neophylidenemolybdenum(VI) (2,6-dimethylphenylimido) and 500 parts of toluene were placed in a glass reaction vessel equipped with a stirrer, and stirred. Then, 80 parts of the cross-linked rubber C (50 parts as the amount of organic polymers), which was obtained in Preparation Example 9 and cut into small pieces with a dimension of 1 mm × 2 mm × 2 mm, was added thereto, and a depolymerization reaction was carried out with stirring at 50°C for 24 hours. The cross-linked rubber C in the form of small pieces with a dimension of 1 mm × 2 mm × 2 mm lost their shape as the depolymerization reaction proceeded, and was dissolved and dispersed in toluene to form black precipitates of carbon. After the reaction, methanol was added in excess to terminate the depolymerization.

When the amount of cyclopentene contained in the solution after the reaction was quantified by gas chromatography, 49.5 parts of cyclopentene was produced (the concentration of cyclopentene in the cyclopentene solution was 1.260 mol/L), and the cyclopentene recovery rate was 99%. No peak corresponding to a high molecular weight was observed by GPC measurement of the soluble part.

### <Example 29>

Under a nitrogen atmosphere, 50 parts of the ring-opened polymer A obtained in Preparation Example 1 and 400 parts of toluene (0.462 L) were placed in a glass reaction vessel equipped with a stirrer, stirred, and dissolved. Then, a catalyst solution, in which 2.86 parts of diethylaluminum ethoxide was added to a solution of 2.90 parts of tungsten hexachloride dissolved in 110 parts of toluene, was added thereto, and a depolymerization reaction was carried out with stirring at 50°C for 24 hours. After the reaction, methanol was added in excess to terminate the depolymerization.

When the amount of cyclopentene contained in the solution after the reaction was quantified by gas chromatography, 15.7 parts of cyclopentene was produced (the concentration of cyclopentene in the cyclopentene solution (cyclopentene solution in toluene) was 0.401 mol/L), and the cyclopentene recovery rate was 83%. The number average molecular weight (Mn) of the ring-opened polymer determined by GPC measurement of the soluble part was 8,900, and the weight average molecular weight (Mw) was 23,300. Further, the ratio of 2-norbornene structural units/cyclopentene structural units determined by ¹H-NMR was 91/9 (weight ratio), which was in good agreement with the result obtained by gas chromatography (i.e., the amount of recovered cyclopentene).

### <Example 30>

Under a nitrogen atmosphere, 50 parts of the ring-opened polymer B obtained in Preparation Example 2 and 400 parts of toluene (0.462 L) were placed in a glass reaction vessel equipped with a stirrer, stirred, and dissolved. Then, a catalyst solution, in which 2.86 parts of diethylaluminum ethoxide was added to a solution of 2.90 parts of tungsten hexachloride dissolved in 110 parts of toluene, was added thereto, and a depolymerization reaction was carried out with stirring at 50°C for 24 hours. After the reaction, methanol was added in excess to terminate the depolymerization.

When the amount of cyclopentene contained in the solution after the reaction was quantified by gas chromatography, 25.9 parts of cyclopentene was produced (the concentration of cyclopentene in the cyclopentene solution (cyclopentene solution in toluene) was 0.660 mol/L), and the cyclopentene recovery rate was 81%. The number average molecular weight (Mn) of the ring-opened polymer determined by GPC measurement of the soluble part was 800, and the weight average molecular weight (Mw) was 3,010. Further, the ratio of dicyclopentadiene structural units/cyclopentene structural units determined by ¹H-NMR was 76/24 (weight ratio), which was in good agreement with the result obtained by gas chromatography (i.e., the amount of recovered cyclopentene).

### <Example 31>

Under a nitrogen atmosphere, 50 parts of the ring-opened polymer C obtained in Preparation Example 3 and 400 parts of toluene (0.462 L) were placed in a glass reaction vessel equipped with a stirrer, stirred, and dissolved. Then, a catalyst solution, in which 2.86 parts of diethylaluminum ethoxide was added to a solution of 2.90 parts of tungsten hexachloride dissolved in 110 parts of toluene, was added thereto, and a depolymerization reaction was carried out with stirring at 50°C for 24 hours. After the reaction, methanol was added in excess to terminate the depolymerization.

When the amount of cyclopentene contained in the solution after the reaction was quantified by gas chromatography, 50.0 parts of cyclopentene was produced (the concentration of cyclopentene in the cyclopentene solution (cyclopentene solution in toluene) was 1.272 mol/L), and the cyclopentene recovery rate was 100%. No peak corresponding to a high molecular weight was observed by GPC measurement of the soluble part.

### <Example 32>

Under a nitrogen atmosphere, a catalyst solution, in which 2.86 parts of diethylaluminum ethoxide was added to a solution of 2.90 parts of tungsten hexachloride dissolved in 510 parts of toluene, and 80 parts of the cross-linked rubber A (50 parts as the amount of organic polymers), which was obtained in Preparation Example 7 and cut into small pieces with a dimension of 1 mm × 2 mm × 2 mm, were placed in a glass reaction vessel equipped with a stirrer, and a depolymerization reaction was carried out with stirring at 50°C for 24 hours. The cross-linked rubber A in the form of small pieces with a dimension of 1 mm × 2 mm × 2 mm lost their shape as the depolymerization reaction proceeded, and was dissolved and dispersed in toluene to form black precipitates of carbon. After the reaction, methanol was added in excess to terminate the depolymerization.

When the amount of cyclopentene contained in the mixture after the reaction was quantified by gas chromatography, 12.0 parts of cyclopentene was produced (the concentration of cyclopentene in the cyclopentene solution was 0.305 mol/L), and the cyclopentene recovery rate was 63%. The number average molecular weight (Mn) of the ring-opened polymer determined by GPC measurement of the soluble part was 72,000, and the weight average molecular weight (Mw) was 187,000. Further, the ratio of 2-norbornene structural units/cyclopentene structural units determined by ¹H-NMR was 78/22 (weight ratio), which was in good agreement with the result obtained by gas chromatography (i.e., the amount of recovered cyclopentene).

### <Example 33>

Under a nitrogen atmosphere, a catalyst solution, in which 2.86 parts of diethylaluminum ethoxide was added to a solution of 2.90 parts of tungsten hexachloride dissolved in 510 parts of toluene, and 80 parts of the cross-linked rubber B (50 parts as the amount of organic polymers), which was obtained in Preparation Example 8 and cut into small pieces with a dimension of 1 mm × 2 mm × 2 mm, were placed in a glass reaction vessel equipped with a stirrer, and a depolymerization reaction was carried out with stirring at 50°C for 24 hours. The cross-linked rubber B in the form of small pieces with a dimension of 1 mm × 2 mm × 2 mm lost their shape as the depolymerization reaction proceeded, and was dissolved and dispersed in toluene to form black precipitates of carbon. After the reaction, methanol was added in excess to terminate the depolymerization.

When the amount of cyclopentene contained in the mixture after the reaction was quantified by gas chromatography, 21.1 parts of cyclopentene was produced (the concentration of cyclopentene in the cyclopentene solution was 0.537 mol/L), and the cyclopentene recovery rate was 66%. The number average molecular weight (Mn) of the ring-opened polymer determined by GPC measurement of the soluble part was 78,500, and the weight average molecular weight (Mw) was 154,000. Further, the ratio of dicyclopentadiene structural units/cyclopentene structural units determined by ¹H-NMR was 61/39 (weight ratio), which was in good agreement with the result obtained by gas chromatography (i.e., the amount of recovered cyclopentene).

### <Example 34>

Under a nitrogen atmosphere, a catalyst solution, in which 2.86 parts of diethylaluminum ethoxide was added to a solution of 2.90 parts of tungsten hexachloride dissolved in 510 parts of toluene, and 80 parts of the cross-linked rubber C (50 parts as the amount of organic polymers), which was obtained in Preparation Example 9 and cut into small pieces with a dimension of 1 mm × 2 mm × 2 mm, were placed in a glass reaction vessel equipped with a stirrer, and a depolymerization reaction was carried out with stirring at 50°C for 24 hours. The cross-linked rubber C in the form of small pieces with a dimension of 1 mm × 2 mm × 2 mm lost their shape as the depolymerization reaction proceeded, and was dissolved and dispersed in toluene to form black precipitates of carbon. After the reaction, methanol was added in excess to terminate the depolymerization.

When the amount of cyclopentene contained in the mixture after the reaction was quantified by gas chromatography, 49.5 parts of cyclopentene was produced (the concentration of cyclopentene in the cyclopentene solution was 1.260 mol/L), and the cyclopentene recovery rate was 99%. No peak corresponding to a high molecular weight was observed by GPC measurement of the soluble part.

### <Comparative Example 1>

In a 1-L vessel, 75 g of nitrile rubber (Perbunan (registered trademark) T 3429) was dissolved in 500 g of monochlorobenzene. After the nitrile rubber was completely dissolved, 3 g of 1-hexene was added to the vessel, and the solution was stirred for 2 hours. During this stirring, 1,3-bis-(2,4,6-trimethylphenyl)-2-imidazolidinylidene) (tricyclohexylphosphine)-ruthenium(phenyl-methylene) dichloride was dissolved in 20 mL of monochlorobenzene and added to the 1-L vessel. The reaction mixture was allowed to react at 22°C for 12 hours with stirring. After this predetermined time, the solution was analyzed by gas chromatography. No monomer such as cyclopentene or butadiene was detected by the gas chromatography analysis.

### <Comparative Example 2>

Under a nitrogen atmosphere, 5 parts of the cross-linked rubber C, which was obtained in Preparation Example 8 and cut into small pieces with a dimension of 1 mm × 2 mm × 2 mm, and 500 parts of toluene were placed in a glass reaction vessel equipped with a stirrer, and stirred. 13.8 Parts of 2,2'-azobis(isobutyronitrile) (AIBN) was added thereto as a deterioration accelerator, and then resulting solution was exposed to ultraviolet light for 5 hours. The irradiation distance was 30 cm. After the reaction, although the viscosity of the solution was drastically decreased, no peak of derived from cyclopentene was observed by gas chromatography analysis.

### [Table 1]

**Table 1**

| | | | Preparation Example | | | | | |
|---|---|---|---|---|---|---|---|---|
| | | | 1 | 2 | 3 | 4 | 5 | 6 |
| | | | Ring-opened polymer A | Ring-opened polymer B | Ring-opened polymer C | Ring-opened polymer D | Ring-opened polymer E | Ring-opened polymer F |
| Raw materials used for polymerization reaction | | | | | | | | |
| | 2-Norbornene | (parts) | 100 | 0 | 0 | 41 | 0 | 0 |
| | Dicyclopentadiene | (parts) | 0 | 72 | 0 | 39 | 0 | 100 |
| | 1,4-Methano-1,4,4a,9a-tetrahydro-9H-fluorene | (parts) | 0 | 0 | 0 | 0 | 65 | 0 |
| | Cyclopentene | (parts) | 100 | 188 | 200 | 100 | 84 | 100 |
| | Toluene | (parts) | 394 | 1160 | 394 | 394 | 400 | 400 |
| | 1-Hexene | (parts) | 0.12 | 0.17 | 0.17 | 0.14 | 0.15 | 0 |
| | Ring-opening polymerization catalyst | (parts) | 0.006 | 0.028 | 0.006 | 0.017 | 0.5 | 0.9 |
| Number average molecular weight (Mn) | | | 122,000 | 120,000 | 115,000 | 126,000 | 121,000 | 157,000 |
| Weight average molecular weight (Mw) | | | 268,000 | 248,000 | 235,000 | 254,000 | 244,000 | 184,000 |
| Weight ratio of norbornene units/cyclopentene units | | | 62/38 | 36/64 | 0/100 | 52/48 | 51/49 | 65/35 |
| Glass transition temperature | | (°C) | -25 | -45 | -100 | -40 | -23 | -100, 150 |

### [Table 2]

**Table 2**

| | Preparation Example | | | | | |
|---|---|---|---|---|---|---|
| | 7 | 8 | 9 | 10 | 11 | 12 |
| | Cross-linked rubber A | Cross-linked rubber B | Cross-linked rubber C | Cross-linked rubber D | Cross-linked rubber E | Cross-linked rubber F |
| Ring-opened polymer used | Ring-opened polymer A | Ring-opened polymer B | Ring-opened polymer C | Ring-opened polymer D | Ring-opened polymer E | Ring-opened polymer F |
| Weight ratio of norbornene units/cyclopentene units of ring-opened polymer | 62/38 | 36/64 | 0/100 | 52/48 | 51/49 | 65/35 |

### [Table 3]

**Table 3**

| | | Example | | | | | | | | |
|---|---|---|---|---|---|---|---|---|---|---|
| | | 1 | 2 | 3 | 4 | 5 | 6 | 7 | 8 | 9 |
| Polymer or cross-linked product used for recovery | | Ring-opened polymer A | Ring-opened polymer B | Ring-opened polymer B | Ring-opened polymer D | Ring-opened polymer E | Cross-linked rubber A | Cross-linked rubber B | Cross-linked rubber B | Cross-linked rubber C |
| Weight ratio of norbornene units/cyclopentene units of ring-opened polymer | | 62/38 | 36/64 | 36/64 | 52/48 | 51/49 | 62/38 | 36/64 | 36/64 | 0/100 |
| Amount of polymer or cross-linked rubber used for recovery | (parts) | 50 | 50 | 50 | 50 | 50 | 80 | 80 | 80 | 80 |
| Amount of organic polymer of polymer or cross-linked rubber used for recovery | (parts) | 50 | 50 | 50 | 50 | 50 | 50 | 50 | 50 | 50 |
| Amount of cyclopentene in polymer or cross-linked rubber used for recovery | (parts) | 19 | 32 | 32 | 24 | 24.5 | 19 | 32 | 32 | 50 |
| Amount of toluene used | (parts) | 500 | 500 | 100 | 500 | 500 | 500 | 500 | 100 | 500 |
| Amount of toluene used (in liters) | (L) | 0.577 | 0.577 | 0.115 | 0.577 | 0.577 | 0.577 | 0.577 | 0.115 | 0.577 |
| Concentration of cyclopentene on assumption that reaction completely occurs and cyclopentene is all recovered | (mol/L) | 0.483 | 0.814 | 4.071 | 0.611 | 0.623 | 0.483 | 0.814 | 4.071 | 1.272 |
| Type of metathesis catalyst | | Ru catalyst | Ru catalyst | Ru catalyst | Ru catalyst | Ru catalyst | Ru catalyst | Ru catalyst | Ru catalyst | Ru catalyst |
| Amount of metathesis catalyst used | (parts) | 0.08 | 0.1 | 0.1 | 0.1 | 0.1 | 1.2 | 1.4 | 1.4 | 0.5 |
| Reaction temperature of depolymerization | (°C) | 25 | 25 | 25 | 25 | 25 | 25 | 25 | 25 | 25 |
| Reaction time of depolymerization | (hr) | 2 | 2 | 2 | 2 | 2 | 2 | 2 | 2 | 2 |
| Amount of recovered cyclopentene | (parts) | 15.2 | 25.6 | 9.3 | 17.9 | 18.4 | 13.1 | 22.5 | 8.6 | 44.3 |
| Cyclopentene recovery rate | (%) | 80 | 80 | 29 | 75 | 75 | 69 | 70 | 27 | 89 |
| Concentration of cyclopentene in cyclopentene solution | (mol/L) | 0.387 | 0.651 | 1.183 | 0.455 | 0.468 | 0.333 | 0.573 | 1.094 | 1.127 |
| Number average molecular weight (Mn) of ring-opened polymer after treatment for recovery | | 71,000 | 18,000 | 99,000 | 45,000 | 42,000 | 90,000 | 66,000 | 99,000 | Not detected |
| Weight average molecular weight (Mw) of ring-opened polymer after treatment for recovery | | 128,000 | 41,000 | 205,000 | 87,000 | 86,000 | 183,000 | 136,000 | 212,000 | Not detected |
| Weight ratio of norbornene units/cyclopentene units of ring-opened polymer after treatment for recovery | | 89/11 | 74/26 | 43/57 | 81/19 | 84/16 | 84/16 | 65/35 | 42/58 | --- |

### [Table 4]

**Table 4**

| | | Example | | | | | | | | | | | | |
|---|---|---|---|---|---|---|---|---|---|---|---|---|---|---|
| | | 10 | 11 | 12 | 12 | 14 | 15 | 16 | 17 | 18 | 19 | 20 | 21 | 22 |
| Polymer or cross-linked product used for recovery | | Cross-linked rubber C | Cross-linked rubber D | Cross-linked rubber E | Cross-linked rubber A | Cross-linked rubber A | Ring-opened polymer F | Cross-linked rubber F | Ring-opened polymer A | Ring-opened polymer B | Ring-opened polymer C | Cross-linked rubber A | Cross-linked rubber B | Cross-linked rubber C |
| Weight ratio of norbornene units/cyclopentene units of ring-opened polymer | | 0/100 | 52/48 | 51/49 | 62/38 | 62/38 | 65/35 | 65/35 | 62/38 | 36/64 | 0/100 | 62/38 | 36/64 | 0/100 |
| Amount of polymer or cross-linked rubber used for recovery | (parts) | 80 | 80 | 80 | 80 | 80 | 80 | 80 | 50 | 50 | 50 | 80 | 80 | 80 |
| Amount of organic polymer of polymer or cross-linked rubber used for recovery | (parts) | 50 | 50 | 50 | 50 | 50 | 50 | 50 | 50 | 50 | 50 | 50 | 50 | 50 |
| Amount of cyclopentene in polymer or cross-linked rubber used for recovery | (parts) | 50 | 24 | 24.5 | 19 | 19 | 18 | 18 | 19 | 32 | 50 | 19 | 32 | 50 |
| Amount of toluene used | (parts) | 100 | 500 | 500 | 500 | 500 | 500 | 500 | 500 | 500 | 500 | 500 | 500 | 500 |
| Amount of toluene used (in liters) | (L) | 0.115 | 0.577 | 0.577 | 0.577 | 0.577 | 0.577 | 0.577 | 0.577 | 0.577 | 0.577 | 0.577 | 0.577 | 0.577 |
| Concentration of cyclopentene on assumption that reaction completely occurs and cyclopentene is all recovered | (mol/L) | 6.361 | 0.611 | 0.623 | 0.483 | 0.483 | 0.451 | 0.451 | 0.483 | 0.814 | 1.272 | 0.483 | 0.814 | 1.272 |
| Type of metathesis catalyst | | Ru catalyst | Ru catalyst | Ru catalyst | W catalyst | W catalyst | Ru catalyst | Ru catalyst | Ru catalyst | Ru catalyst | Ru catalyst | Ru catalyst | Ru catalyst | Ru catalyst |
| Amount of metathesis catalyst used | (parts) | 0.5 | 1.4 | 1.4 | 4.4 | 5 | 0.1 | 0.5 | 6.23 | 6.23 | 6.23 | 6.23 | 6.23 | 8.23 |
| Reaction temperature of depolymerization | (°C) | 25 | 25 | 25 | 25 | 25 | 25 | 25 | 80 | 80 | 60 | 60 | 80 | 60 |
| Reaction time of depolymerization | (hr) | 2 | 2 | 2 | 2 | 2 | 2 | 2 | 24 | 24 | 24 | 24 | 24 | 24 |
| Amount of recovered cyclopentene | (parts) | 11.5 | 15.8 | 14.6 | 11.6 | 11.4 | 17.2 | 14.1 | 18.2 | 31.7 | 50.0 | 15.2 | 25.6 | 49.5 |
| Cyclopentene recovery rate | (%) | 23 | 66 | 60 | 61 | 60 | 97 | 79 | 96 | 99 | 100 | 80 | 80 | 99 |
| Concentration of cyclopentene in cyclopentene solution | (mol/L) | 1.463 | 0.402 | 0.372 | 0.295 | 0.290 | 0.438 | 0.359 | 0.464 | 0.806 | 1.272 | 0.387 | 0.651 | 1.260 |
| Number average molecular weight (Mn) of ring-opened polymer after treatment for recovery | | 86,000 | 75,000 | 82,000 | 96,000 | 98,000 | 105,000 | 117,000 | 7,100 | 810 | Not detected | 7,760 | 810 | Not detected |
| Weight average molecular weight (Mw) of ring-opened polymer after treatment for recovery | | 176,000 | 158,000 | 141,000 | 184,000 | 189,000 | 123,000 | 149,000 | 15,600 | 2,460 | Not detected | 15,600 | 3,590 | Not detected |
| Weight ratio of norbornene units/cyclopentene units of ring-opened polymer after treatment for recovery | | 0/100 | 76/24 | 72/28 | 80/20 | 79/21 | 100/0 | 91/9 | 98/2 | 99/1 | - | 89/11 | 75/25 | - |

### [Table 5]

**Table 5**

| | | Example | | | | | | | | | | | | Comp. Ex. | |
|---|---|---|---|---|---|---|---|---|---|---|---|---|---|---|---|
| | | 23 | 24 | 25 | 26 | 27 | 28 | 29 | 30 | 31 | 32 | 33 | 34 | 1 | 2 |
| Polymer or cross-linked product used for recovery | | Ring-opened polymer A | Ring-opened polymer B | Ring-opened polymer C | Cross-linked rubber A | Cross-linked rubber B | Cross-linked rubber C | Ring-opened polymer A | Ring-opened polymer B | Ring-opened polymer C | Cross-linked rubber A | Cross-linked rubber B | Cross-linked rubber C | NBR | Cross-linked rubber C |
| Weight ratio of norbornene units/cyclopentene units of ring-opened polymer | | 62/38 | 36/64 | 0/100 | 62/38 | 36/64 | 0/100 | 62/36 | 36/64 | 0/100 | 62/38 | 36/64 | 0/100 | - | 0/100 |
| Amount of polymer or cross-linked rubber used for recovery | (parts) | 50 | 50 | 50 | 80 | 80 | 80 | 50 | 50 | 50 | 80 | 80 | 80 | - | - |
| Amount of organic polymer of polymer or cross-linked rubber used for recovery | (parts) | 50 | 50 | 50 | 50 | 50 | 50 | 50 | 50 | 50 | 50 | 50 | 50 | 75 | 5 |
| Amount of cyclopentene in polymer or cross-linked rubber used for recovery | (parts) | 19 | 32 | 50 | 19 | 32 | 50 | 19 | 32 | 50 | 19 | 32 | 50 | 0 | 5 |
| Amount of toluene used | (parts) | 500 | 500 | 500 | 500 | 500 | 500 | 500 | 500 | 500 | 500 | 500 | 500 | 500 | 500 |
| Amount of toluene used (in liters) | (L) | 0.577 | 0.577 | 0.577 | 0.577 | 0.577 | 0.577 | 0.577 | 0.577 | 0.577 | 0.577 | 0.577 | 0.577 | - | - |
| Concentration of cyclopentene on assumption that reaction completely occurs and cyclopentene is all recovered | (mol/L) | 0.483 | 0.814 | 1.272 | 0.483 | 0.814 | 1.272 | 0.483 | 0.814 | 1.272 | 0.483 | 0.814 | 1.272 | - | - |
| Type of metathesis catalyst | | Mo catalyst | Mo catalyst | Mo catalyst | Mo catalyst | Mo catalyst | Mo catalyst | W catalyst | W catalyst | W catalyst | W catalyst | W catalyst | W catalyst | Ru catalyst | AlBN was used |
| Amount of metathesis catalyst used | (parts) | 0.21 | 0.21 | 0.21 | 5.43 | 5.43 | 5.43 | 2.9 | 2.8 | 2.8 | 2.9 | 2.8 | 2.9 | 20mL | |
| Reaction temperature of depolymerization | (°C) | 25 | 25 | 25 | 50 | 50 | 50 | 50 | 50 | 50 | 50 | 50 | 50 | 22 | UV irradiation |
| Reaction time of depolymerization | (hr) | 2 | 2 | 2 | 24 | 24 | 24 | 24 | 24 | 24 | 24 | 24 | 24 | 12 | |
| Amount of recovered cyclopentene | (parts) | 13.3 | 24 | 500 | 14.4 | 25.6 | 49.5 | 157 | 25.9 | 50.0 | 12 | 21.1 | 49.5 | Not recoverable | Not recoverable |
| Cyclopentene recovery rate | (%) | 70 | 75 | 100 | 76 | 80 | 99 | 83 | 81 | 100 | 63 | 66 | 99 | | |
| Concentration of cyclopentene in cyclopentene solution | (mol/L) | 0.338 | 0.611 | 1.272 | 0.367 | 0.651 | 1.260 | 0.401 | 0.660 | 1.272 | 0.305 | 0.537 | 1.260 | | |
| Number average molecular weight (Mn) of ring-opened polymer after treatment for recovery | | 83,000 | 55,000 | Not detected | 16,700 | 820 | Not detected | 8,900 | 800 | Not detected | 72,000 | 78,500 | Not detected | | |
| Weight average molecular weight (Mw) of ring-opened polymer after treatment for recovery | | 139,000 | 97,000 | Not detected | 33,900 | 4,200 | Not detected | 23,300 | 3,010 | Not detected | 187,000 | 154,000 | Not detected | | |
| Weight ratio of norbornene units/cyclopentene units of ring-opened polymer after treatment for recovery | | 85/15 | 70/30 | --- | 86/14 | 75/25 | --- | 91/9 | 76124 | --- | 76/22 | 61/39 | --- | | |

In Tables 1 to 5, the term "weight ratio of norbornene units/cyclopentene units" refers to a weight ratio between units derived from norbornene compounds (2-norbornene, dicyclopentadiene, and 1,4-methano-1,4,4a,9a-tetrahydro-9H-fluorene) and units derived from cyclopentene.

## Claims

1. A method for recovering cyclopentene, comprising allowing a metathesis catalyst to act on a ring-opened polymer having a structural unit derived from cyclopentene to recover cyclopentene.

2. The method for recovering cyclopentene according to claim 1, wherein the ring-opened polymer is a cyclopentene homopolymer or a copolymer of cyclopentene and a norbornene compound.

3. The method for recovering cyclopentene according to claim 1 or 2, wherein the ring-opened polymer is a copolymer of cyclopentene and a norbornene compound.

4. The method for recovering cyclopentene according to claim 2 or 3, wherein the ring-opened polymer is a random copolymer of cyclopentene and a norbornene compound.

5. The method for recovering cyclopentene according to any one of claims 1 to 4, wherein the ring-opened polymer is a cross-linked polymer.

6. The method for recovering cyclopentene according to any one of claims 1 to 5, wherein cyclopentene is recovered by allowing the metathesis catalyst to act on the ring-opened polymer in a solvent while controlling the concentration of cyclopentene to be in a range of 1.7 mol/L or less.

7. The method for recovering cyclopentene according to any one of claims 1 to 6, wherein cyclopentene is recovered by allowing the metathesis catalyst to act on the ring-opened polymer in a solvent while controlling the amount of the ring-opened polymer used such that the concentration of polycyclopentene repeating units in the ring-opened polymer is 5 mol/L or less.

8. A cyclopentene solution containing cyclopentene and a reaction residue of a metathesis catalyst in a solvent, wherein the concentration of cyclopentene is 0.01 to 1.7 mol/L.
